(19) Europäisches Patentamt
European Patent Office
Office européen des brevets

(11) EP 1 410 785 B1

(12) # FASCICULE DE BREVET EUROPEEN

(45) Date de publication et mention de la délivrance du brevet:
**03.01.2007 Bulletin 2007/01**

(51) Int Cl.:
***A61Q 1/02*** *(2006.01)* ***A61K 8/49*** *(2006.01)*

(21) Numéro de dépôt: **03292569.5**

(22) Date de dépôt: **15.10.2003**

(54) **Produit de maquillage associant deux compositions à base respectivement d'une matière colorante photochrome et d'un agent goniochromatique**

Schminkmittel, vereinend zwei Zusammensetzungen, die eine enthaltend einen photochromen Farbstoff, die andere ein goniochromatisches Agens

Make-up product, combining two compositions, comprising a photochromic dye and a goniochromatic agent, respectively

(84) Etats contractants désignés:
**AT BE BG CH CY CZ DE DK EE ES FI FR GB GR HU IE IT LI LU MC NL PT RO SE SI SK TR**

(30) Priorité: **18.10.2002 FR 0213035**
**18.10.2002 FR 0213037**

(43) Date de publication de la demande:
**21.04.2004 Bulletin 2004/17**

(73) Titulaire: **L'ORÉAL**
**75008 Paris (FR)**

(72) Inventeurs:
- **Simon, Jean-Christophe**
  **162-0842 Tokyo (JP)**
- **Blin, Xavier**
  **75015 Paris (FR)**

(74) Mandataire: **Le Coupanec, Pascale A.M.P.**
**Nony & Associés,**
**3 rue de Penthièvre**
**75008 Paris (FR)**

(56) Documents cités:
**EP-B- 0 973 761**
**FR-A- 2 814 677**
**FR-A- 2 815 848**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



EP 1 410 785 B1

## Description

La présente invention se rapporte à un nouveau produit cosmétique notamment de maquillage de la peau, des lèvres et/ou des phanères associant au moins un agent de coloration goniochromatique et au moins une matière colorante photochrome.

Par « produit de maquillage », on entend un produit contenant une matière colorante permettant le dépôt d'une couleur sur une matière kératinique (la peau, les lèvres ou les phanères) de personne humaine telle qu'un rouge à lèvres, un fard, un eye-liner, un fond de teint, un autobronzant ou encore un produit de maquillage ayant des propriétés de soin ou un produit de maquillage semi-permanent (tatouage).

Plus particulièrement, le produit considéré selon l'invention comprend au moins deux compositions cosmétiques qui peuvent être appliquées successivement sur la peau aussi bien du visage que du corps humain, sur les paupières inférieures et supérieures des êtres humains, sur les lèvres et sur les phanères comme les ongles, les sourcils, les cils ou les cheveux, ainsi qu'un procédé de maquillage bicouche du visage et du corps humain.

Par composition cosmétique, on désigne une composition telle que définie dans la directive 93/35/CEE du conseil du 14 juin 1993.

L'une des fonctions principales du maquillage consiste à apporter des effets de couleurs et/ou optiques au niveau des zones à maquiller sur la peau, les lèvres, les cils et les ongles.

D'une manière générale, ces effets sont statiques, c'est-à-dire n'évoluent ni au cours du temps, ni en fonction de la luminosité ambiante. L'évolution la plus fréquente consiste généralement en une altération progressive dans le temps des effets de couleurs et/ou optique d'origine. Cette altération est généralement due soit à un transfert et/ou une migration de la composition, phénomènes particulièrement prononcés pour les rouges à lèvres, soit à une mauvaise tenue des pigments présents dans la composition de maquillage vis-à-vis du sébum, phénomène plus particulièrement rencontré avec les fonds de teint.

Il n'existe donc pas à ce jour de produit de maquillage que l'on pourrait qualifier de dynamique en terme de teinte, c'est-à-dire susceptible de se modifier significativement en terme de couleur et/ou d'éclat, en réponse par exemple à une modification de la luminosité ambiante.

Or, il est clair que ce type de produit de maquillage répondrait précisément à un souhait des consommatrices qui sont à la recherche de maquillages colorés originaux contrastant avec des compositions de maquillage conventionnelles.

L'invention a précisément pour objectif principal de répondre à ce besoin. Plus particulièrement, elle vise à proposer un produit cosmétique doté d'un effet couleur et/ou optique dit dynamique.

En l'occurrence, la présente invention met notamment à profit la faculté manifestée par certaines matières colorantes photochromes à changer de couleur dès qu'elles sont soumises à un rayonnement contenant des rayons ultraviolets et ceci d'une manière réversible.

L'utilisation de pigments photochromes dans des compositions cosmétiques est connue. La demande EP 970 689 décrit un produit bi-couche de maquillage permettant de superposer à une couche de base contenant un pigment photochrome, une couche de surface contenant un filtre de lumière ultraviolette. Le maquillage correspondant permet de former des motifs qui apparaissent ou disparaissent selon la nature de la lumière mais il ne lui est associé aucune dynamique en terme de couleur. La demande WO 02/078665 propose pour sa part des compositions cosmétiques comprenant des agents photochromes de type naphtopyrane. Toutefois, ceux-ci sont incorporés dans la composition cosmétique sous une forme liquide encapsulée dans une microcapsule. Cette forme dispersible est décrite comme dotée d'une activité anti-oxydante.

D'une manière inattendue, les inventeurs ont constaté qu'il était particulièrement avantageux d'associer au moins une matière colorante goniochromatique à au moins une matière colorante photochrome spécifique pour obtenir un effet dynamique original en terme de couleur et d'éclat.

En l'occurrence, la présente invention concerne, selon un premier aspect, un produit cosmétique notamment de maquillage de la peau, des lèvres et/ou des phanères comprenant au moins une première et une seconde compositions, la première composition comprenant, dans un premier milieu physiologiquement acceptable, au moins une matière colorante dite première matière colorante et la seconde composition comprenant dans un second milieu physiologiquement acceptable, au moins une matière colorante dite seconde matière colorante, ladite première matière colorante étant photochrome et ladite seconde matière colorante étant au moins un agent de coloration goniochromatique.

Selon une variante particulière, ladite première matière colorante photochrome est de type naphtopyrane.

La présente invention concerne également selon un deuxième aspect, un produit cosmétique notamment de maquillage de la peau, des lèvres et/ou des phanères comprenant au moins une première et une seconde compositions, la première composition comprenant, dans un premier milieu physiologiquement acceptable, au moins une matière colorante dite première matière colorante et la seconde composition comprenant dans un second milieu physiologiquement acceptable, au moins une matière colorante dite seconde matière colorante, ladite première matière colorante étant de type naphtopyrane, et ladite seconde matière colorante étant au moins un agent de coloration goniochromatique.

Avantageusement, l'association de ladite première matière colorante conforme à l'invention, c'est-à-dire photochrome et/ou de type naphtopyrane à au moins un agent de coloration goniochromatique dans un produit cosmétique plus particulièrement destiné au maquillage permet de conférer à ce dernier une dynamique rapide et réversible en terme d'effet de teinte et/ou d'éclat. Ainsi, l'écart de teinte est suffisamment significatif pour être remarqué à l'oeil nu. D'autre part, il est immédiat, c'est-à-dire se manifeste dans un délai très court, après soit l'exposition du maquillage à un rayonnement UV, soit arrêt de cette exposition.

Outre cet aspect « effet de teinte et/ou d'éclat dynamique », les produits selon l'invention sont tout particulièrement avantageux pour suppléer au phénomène d'altération des couleurs qui a été discuté précédemment et qui est généralement lié à une mauvaise tenue des pigments conventionnels au sébum. L'association à ces pigments d'une première matière colorante conforme à l'invention permet en effet avantageusement de compenser ce type d'altération dans la mesure où ladite matière colorante est capable à elle seule, en réponse à une excitation lumineuse de type UV, de restituer un effet coloré équivalent à celui procuré initialement par lesdits pigments.

Comme précisé précédemment, le produit selon l'invention comprend deux (ou plusieurs) compositions physiologiquement acceptables.

Les compositions peuvent être conditionnées séparément ou ensemble dans un même article de conditionnement ou dans deux (ou plusieurs) articles de conditionnement séparés ou distincts. Chaque composition peut être une poudre libre ou compactée, un fond de teint, un fard à joues ou à paupières, un produit anti-cerne, un blush, un rouge à lèvres, un baume à lèvres, un brillant à lèvres, un crayon à lèvres ou à yeux, un mascara, un eye-liner, un vernis à ongles ou encore un produit de maquillage du corps ou de coloration de la peau. En l'occurrence, la première composition du produit selon l'invention peut constituer une couche de base appliquée sur le support à maquiller, notamment les lèvres et la seconde composition, une couche de dessus (ou « top coat »). Il est toutefois possible d'appliquer, sous la première couche, une sous couche ayant la constitution ou non de la seconde composition. Il est également possible de déposer une surcouche sur la seconde couche ayant une constitution identique ou non à celle de la première couche.

L'invention concerne selon un autre de ses aspects, un kit de maquillage contenant un produit cosmétique de maquillage tel que défini précédemment, dans lequel les différentes compositions peuvent être notamment conditionnées séparément et sont accompagnées de moyens d'application appropriés. Ces moyens peuvent être des pinceaux, des brosses, des stylos, des crayons, des feutres, des plumes, des éponges et/ou des mousses.

L'invention se rapporte aussi selon un autre de ses aspects, à un procédé de maquillage de la peau, des lèvres et/ou des phanères consistant à appliquer sur la peau, les lèvres et/ou les phanères un produit cosmétique de maquillage tel que défini précédemment.

Plus particulièrement, on applique, en une première couche, la première composition comprenant dans un premier milieu physiologiquement acceptable au moins une matière colorante, dite première matière colorante, puis l'on applique, sur tout ou partie de ladite première couche, une couche de la seconde composition comprenant, dans un second milieu physiologiquement acceptable, au moins un agent de coloration goniochromatique, dit encore seconde matière colorante.

En particulier, la première matière colorante est photochrome et/ou de type naphtopyrane.

De préférence, le maquillage obtenu est un maquillage bicouche. En particulier, la couche de base est un fond de teint, un fard, un rouge à lèvres, un brillant à lèvres, un eye-liner, un produit de maquillage du corps, et la couche du dessus ou « top coat » un produit de soin ou de protection.

De préférence, on laisse sécher la première couche de la première composition dite couche de base, avant d'appliquer la seconde couche de la seconde composition.

L'ordre de superposition, et/ou le mode de superposition des deux compositions à savoir total ou partiel, peut par ailleurs être intéressant pour conférer des effets visuels supplémentaires. La seconde couche peut par exemple être disposée de manière à former des motifs, à la surface de la couche de base. Ainsi, lorsque la couche du dessus (top coat) contient la matière colorante photochrome, il est possible d'appliquer la seconde couche sur une partie seulement de la première couche contenant l'agent goniochromatique. Seule la partie sur laquelle sont superposées les deux couches sera susceptible de présenter un effet de couleur en absence de rayonnement UV.

Ce maquillage bicouche peut être adapté pour tous les produits de maquillage de la peau aussi bien du visage que du scalp et du corps d'êtres humains, des muqueuses comme les lèvres, de l'intérieur des paupières inférieures, et des phanères comme les ongles, les cils, les cheveux, les sourcils, voire les poils.

## MATIERE COLORANTE PHOTOCHROME

D'une manière générale, une matière colorante photochrome est une matière colorante ayant la propriété de changer de teinte lorsqu'elle est éclairée par de la lumière ultraviolette et de rétablir sa couleur initiale lorsqu'elle n'est plus éclairée par cette lumière ou encore de passer d'un état non coloré à un état coloré et inversement. En d'autres termes, une telle matière présente des teintes différentes selon qu'elle est éclairée par de la lumière contenant un certaine quantité de radiations UV comme la lumière solaire ou de la lumière artificielle.

Au sens de la présente invention, « matière colorante photochrome » désigne une matière colorante se caractérisant par un écart de teinte ΔE au moins égal à 5.

Au sens de la présente invention, ΔE figure l'écart de teinte observé au niveau de la matière photochromique entre son état excité, c'est-à-dire en présence d'irradiation UV et son état non excité, c'est-à-dire en absence d'irradiation UV.

Le ΔE peut être déterminé à partir de l'espace chromatique et plus particulièrement à partir des coordonnées chromatiques spécifiques de la matière colorante photochrome considérée, évaluées d'une part au terme de 2 minutes d'exposition à une radiation UV, généralement à l'aide d'une lumière artificielle contenant un rayonnement UV, et d'autre part au repos, c'est-à-dire 5 secondes après l'arrêt de la radiation.

Plus précisément, le protocole de mesure est le suivant :

- 1 % en poids de la matière colorante photochrome considérée est formulée dans 100 % en poids d'une base blanche de rouge à lèvres de la composition suivante :

- octyl-2 dodecanol 0,5 %
- hectorite modifiée par chlorure de di-stearyl di-methyl ammonium 0,6 %
- lanoline liquide 27,2 %
- cire microcristalline 10,5 %
- cire d'abeille polyglycérolée (3 moles) 4,2 %
- lanoline acétylée 6,7 %
- huile d'arara (esters d'acide oléique) 13,5 %
- cire de lanoline oxypropylénée (5 op) 6,7 %
- érucate d'oléyle 13,5 %
- triglycérides d'acides oléique-linoléique-linolénique 1,7%
- triglycérides d'acides palmitique-oléique-linoléique 13,5 %
- hyaluronate de sodium 0,1 %
- conservateurs 0,1 %
- vitamine 0,5 %
- filtre UV 0,7 %

Sur une carte de contraste de marque LENETA et de référence FORM 1A PENOPAC, on étale une couche de 50 μm d'épaisseur de la composition, à l'aide d'un étaleur automatique. La couche recouvre au moins le fond blanc de la carte.

Les mesures de réflexion sont effectuées à l'aide d'un spectrocolorimètre MINOLTA 3700D. On détermine ainsi les coordonnées trichromatiques initiales de la composition avant irradiation ($L_o$, $a_o$ et $b_o$). On soumet ensuite la composition à un flux de 2 mW/cm$^2$ d'un rayonnement UVA pendant deux minutes puis aussitôt après l'arrêt de l'irradiation, on détermine les nouvelles coordonnées trichromatiques (L, a et b). Il s'écoule moins de 5 secondes entre l'arrêt de l'irradiation et la détermination des nouvelles coordonnées.

Le ΔE est calculé de la manière suivante :

$$\Delta E = [(L - L_o)^2 + (a - a_o)^2 + (b - b_o)^2]^{1/2}$$

Avantageusement, les matières colorantes photochromes selon l'invention possèdent un ΔE au moins égal à 5, notamment supérieur ou égal à 10, en particulier supérieur ou égal à 25 plus particulièrement supérieur ou égal à 35 voire supérieur ou égal à 45.

Une mesure de ΔE supérieure à 30 environ signifie que la matière colorante photochrome donne une couleur vive.

Dans le cadre de la présente invention, les matières colorantes photochromes ont par ailleurs pour avantage de répondre à bref délai soit à une excitation par de la lumière UV soit à un arrêt d'une telle excitation. Il s'en suit une modification rapide en terme de coloration. Cette modification de teinte peut ainsi se traduire dans un laps de temps avantageusement inférieur ou égal à 2 minutes, notamment inférieur ou égal à 1 minute et en particulier inférieur ou égal à 50 secondes.

Selon une variante de l'invention, le produit selon l'invention contient au moins deux matières colorantes photochromes telles que définies ci-dessus, notamment au sein d'une même composition, en association ou non à une matière colorante non photochrome.

La matière colorante photochrome considérée selon l'invention peut être présente à une quantité variant de

0,001 à 20 % en poids, notamment de 0,005 à 10 % en poids, en particulier de 0,01 à 5 % en poids, et plus particulièrement de 0,05 à 2 % en poids, voire de 0,1 à 1 % en poids, par rapport au poids total de la composition la contenant.

Les matières colorantes photochromes selon l'invention peuvent être présentes sous une forme solubilisée ou dispersée au sein de la première ou seconde composition. Selon une variante particulière, elles y sont présentes sous une forme solubilisée.

Plus particulièrement, les matières colorantes photochromes considérées selon l'invention sont de nature organique.

Plus précisément, les matières colorantes organiques photochromes selon l'invention sont des naphtopyranes et notamment tels que définis ci-après.

**MATIERES COLORANTES DE TYPE NAPHTOPYRANE**

Les produits selon l'invention peuvent comprendre une première composition comprenant, à titre de première matière colorante, au moins un dérivé naphtopyrane. Ce dérivé peut être plus particulièrement choisi parmi des 3H-naphto-[2,1-b]-pyranes qui peuvent être représentés par la formule (I) ou des 2H-naphto-[1,2-b]-pyranes qui peuvent être représentés par la formule (II) :

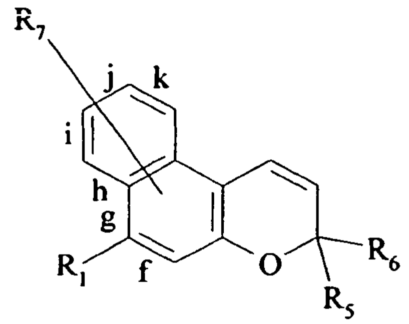

(I)

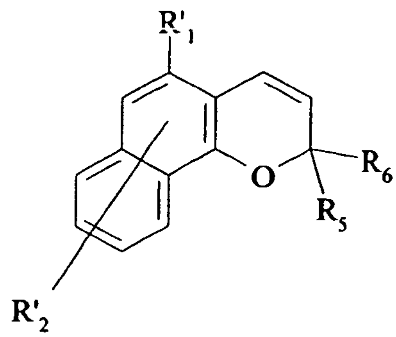

(II)

dans lesquelles :

- R1 représente :

  - (i) un atome d'hydrogène;
  - (ii) un groupement hydrocarboné ayant 1 à 30, notamment 1 à 18, et en particulier 1 à 12, voire 1 à 6 atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné, notamment par F, Br et/ou Cl;
  - (iii) un cycle hydrocarboné formé avec l'une des liaisons "f" ou "gh" et le radical $R_7$; ou
  - (iv) un groupement choisi parmi $-COOR_4$, $-C(O)NR_2R_3$, $-NR_2R_3$, $-OR_4$ et $-SR_4$, dans lequel :

- $R_2$ et $R_3$ soit représentent, indépendamment l'un de l'autre, un groupement hydrocarboné ayant 1 à 20, notamment 1 à 12, et en particulier 1 à 6 atomes de carbone, linéaire, ramifié ou cyclique, saturé ou insaturé, éventuellement comportant 1 à 5 hétéroatomes choisis parmi N, O, S, Si et P,
  soit pris ensemble avec l'atome d'azote auquel ils sont reliés, forment un hétérocycle hydrocarboné, saturé ou insaturé, comportant 3 à 10, notamment 4 à 6 atomes de carbone et éventuellement 1 à 5 autres hétéroatomes choisis parmi N, O, S, Si et P, ledit cycle étant éventuellement substitué par au moins un radical hydrocarboné ayant 1 à 20, notamment 1 à 15, et en particulier 1 à 6 atomes de carbone, linéaire, ramifié ou cyclique, saturé ou insaturé, comportant éventuellement 1 à 5 hétéroatomes choisis parmi N, O, S, Si et P;
- $R_4$ représente un groupement hydrocarboné ayant 1 à 20, notamment 1 à 12, et en particulier 1 à 6 atomes de carbone, linéaire, ramifié ou cyclique, saturé ou insaturé, éventuellement halogéné ou perhalogéné, notamment par F, Br et/ou Cl, et/ou éventuellement comportant 1 à 5 hétéroatomes choisis parmi N, O, S, Si et P;
- $R_5$ et $R_6$ représentent, indépendamment l'un de l'autre, un groupement choisi parmi :

  - (i) les groupements aminoaryles cycliques saturés de formule (IIA) ou (IIB) :

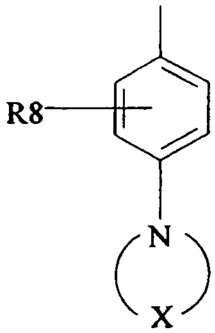

(IIA)

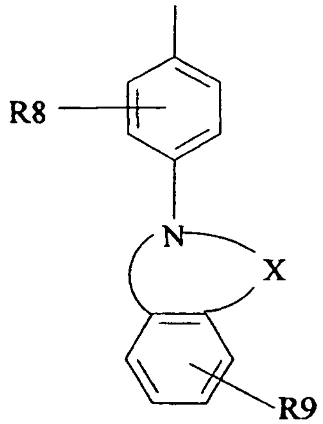

(IIB)

dans lesquelles le cycle comportant N et X est un cycle saturé qui comprend au total 3 à 30 atomes, notamment 4 à 10 et en particulier 5 à 8, voire 5, 6 ou 7 atomes, inclus l'azote, le reste étant des atomes de carbone et/ou des hétéroatomes choisis parmi O, S, Si, P et/ou des groupements choisis parmi -NH et -NR avec R représentant un radical hydrocarboné ayant 1 à 20, notamment 1 à 15, et en particulier 1 à 6 atomes de carbone, linéaire, ramifié ou cyclique, saturé ou insaturé, éventuellement comportant 1 à 5 hétéroatomes choisis parmi N, O, S, Si et P;

- (ii) les groupements indolinoaryles de formule (III) :

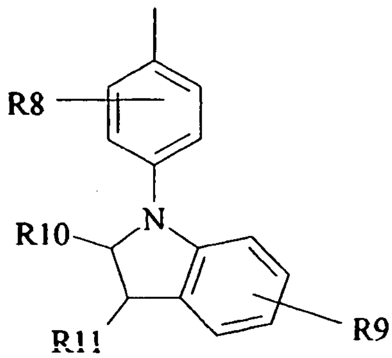

(III)

dans laquelle $R_{10}$ et $R_{11}$ représentent, indépendamment l'un de l'autre, un groupement choisi parmi (i) les groupements hydrocarbonés ayant 1 à 30, notamment 1 à 18, et en particulier 1 à 12, voire 1 à 6 atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné, notamment par F, Br et/ou Cl; (ii) les atomes d'halogène, et notamment F, Br et/ou Cl; (iii) les groupements -CN (nitrile), -COOH (carboxylate), -NO2 (nitro); (iv) un atome d'hydrogène; (v) un groupement choisi parmi $-C(O)NR_2R_3$, $-NR_2R_3$, $-OR_4$ ou $-SR_4$ avec $R_2$, $R_3$ et $R_4$ ayant les significations données ci-dessus; (vi) les radicaux $R_{10}$ et $R_{11}$ pouvant former ensemble un cycle hydrocarboné saturé ou insaturé ayant au total 5 à 8 atomes (incluant les atomes du cycle indoline), lesdits atomes étant choisis parmi C, O, S et/ou NR avec R représentant H ou un radical hydrocarboné ayant 1 à 20, voire 1 à 12 atomes de carbone, linéaire, ramifié ou cyclique, saturé ou insaturé, éventuellement comportant 1 à 5 hétéroatomes choisis parmi N, O, S, Si et P,

- (iii) les groupements de formule (IV) :

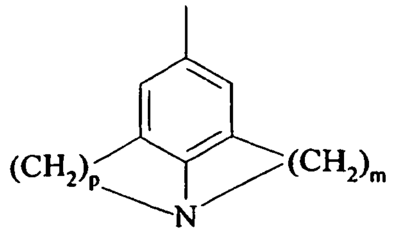

(IV)

dans laquelle m et p sont, indépendamment l'un de l'autre, des entiers allant de 2 à 5;

- (iv) les groupements aminoaryles cycliques insaturés de formules (VA), (VB) ou (VC) :

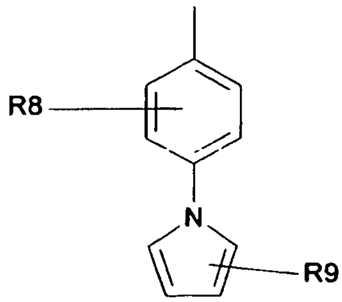

(VA)

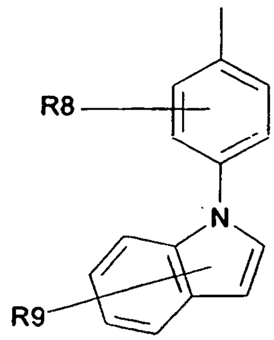

(VB)

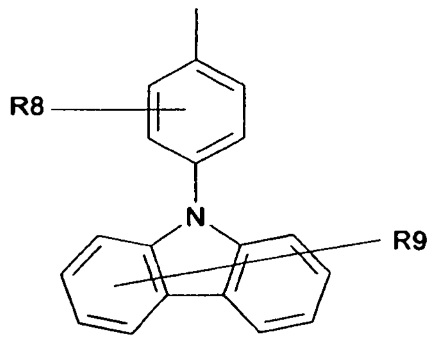

(VC)

dans lesquelles $R_8$ et $R_9$, représentent, indépendamment l'un de l'autre, un groupement choisi parmi (i) les groupements hydrocarbonés ayant 1 à 30, notamment 1 à 18, et en particulier 1 à 12, voire 1-6 atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné, notamment par F, Br et/ou Cl; (ii) les atomes d'halogène, et notamment F, Br et/ou Cl; (iii) les groupements -CN (nitrile), -COOH (carboxylate), $-NO_2$ (nitro); (iv) un atome d'hydrogène; (v) un groupement choisi parmi $-C(O)NR_2R_3$, $-NR_2R_3$, $-OR_4$ ou $-SR_4$ avec $R_2$, $R_3$ et $R_4$ ayant les significations données ci-dessus;

- (v) un groupement hydrocarboné ayant 1 à 30, notamment 2 à 18, et en particulier 3 à 12 atomes de carbone,

linéaire, ramifié ou cyclique, saturé ou insaturé, éventuellement comportant 1 à 5 hétéroatomes choisis parmi N, O, S, Si et P; et notamment un groupement choisi parmi $-C_6H_4-CONR_2R_3$, $-C_6H_4-NR_2R_3$ et $-C_6H_4-OR_4$ avec $R_2$, $R_3$ et $R_4$ ayant les significations données ci-dessus;

- $R_7$ représente un groupement choisi parmi :
  - (i) les groupements hydrocarbonés ayant 1 à 30, notamment 1 à 18, et en particulier 1 à 12 atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné, notamment par F, Br et/ou Cl;
  - (ii) les atomes d'halogène, et notamment F, Br et/ou Cl;
  - (iii) les groupements -CN (nitrile), -COOH (carboxylate), $-NO_2$ (nitro); - N=N- (azo); =NH (imino); $-CONH_2$ (amide);
  - (iv) un atome d'hydrogène;
  - (v) un groupement choisi parmi $-C(O)NR_2R_3$, $-NR_2R_3$, $-OR_4$ ou $-SR_4$ avec $R_2$, $R_3$ et $R_4$ ayant les significations données ci-dessus;
  - (vi) le radical $R_7$ pouvant en outre former, avec l'une des liaisons "i", "j ", "k", ou "g,h" prises avec le radical $R_1$, ou "f" prise avec le radical $R_1$, un cycle hydrocarboné saturé ayant au total 3 à 8, notamment 4 à 7, et en particulier 5 ou 6, atomes de carbone, éventuellement comportant 1 à 5 hétéroatomes choisis parmi N, O, S, Si et P;

- $R'_1$ représente un groupement choisi parmi :
  - un atome d'hydrogène;
  - (ii) un groupement hydrocarboné ayant 1 à 30, notamment 1 à 18, et en particulier 1 à 12 atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné, notamment par F, Br et/ou Cl;
  - (iii) un groupement choisi parmi $-C(O)NR_2R_3$, $-NR_2R_3$, $-OR_4$ et $-SR_4$, avec $R_2$, $R_3$ et $R_4$ ayant les significations données ci-dessus;

- $R'_2$ représente un groupement choisi parmi :
  - (i) les groupements hydrocarbonés ayant 1 à 30, notamment 1 à 18, et en particulier 1 à 12 atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné, notamment par F, Br et/ou Cl;
  - (ii) les atomes d'halogène, et notamment F, Br et/ou Cl;
  - (iii) les groupements -CN (nitrile), -COOH (carboxylate), $-NO_2$ (nitro); -N=N- (azo); =NH (imino); $-CONH_2$ (amide);
  - (iv) un atome d'hydrogène;
  - (v) un groupement choisi parmi $-C(O)NR_2R_3$, $-NR_2R_3$, $-OR_4$ ou $-SR_4$ avec $R_2$, $R_3$ et $R_4$ ayant les significations données ci-dessus.

Notamment, les matières colorantes organiques photochromes selon l'invention peuvent répondre à l'une des formules (Ia) et (IIa) suivantes :

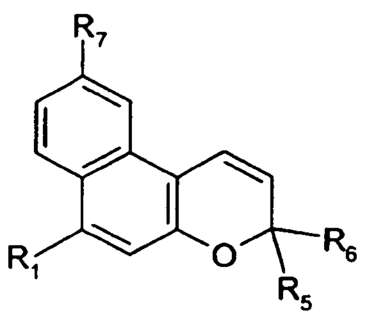

(Ia)

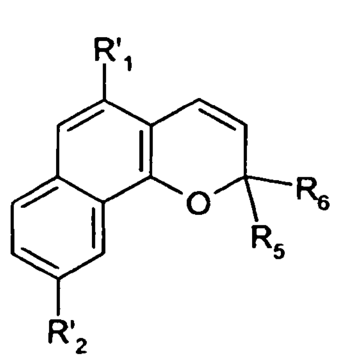

(IIa)

dans lesquelles $R_1$, $R_5$, $R_6$, $R_7$, $R'_1$ et $R'_2$ sont définis tels que précédemment.

En particulier, $R_1$ peut représenter un atome d'hydrogène; un cycle hydrocarboné avec l'une des liaisons "f" ou "gh" et le radical $R_7$; ou un groupement choisi parmi $-COOR_4$, $-NR_2R_3$, $-OR_4$ et $-SR_4$, dans lequel :

- $R_2$ et $R_3$ soit peuvent représenter, indépendamment l'un de l'autre, un groupement hydrocarboné ayant 1 à 20, notamment 1 à 12, et en particulier 1 à 6 atomes de carbone, linéaire, ramifié ou cyclique, saturé ou insaturé, éventuellement comportant 1 à 5 hétéroatomes choisis parmi N, O, S, Si et P,
  soit pris ensemble avec l'atome d'azote auquel ils sont reliés, peuvent former un hétérocycle hydrocarboné, saturé ou insaturé, comportant 3 à 10, notamment 4 à 6 atomes de carbone et éventuellement 1 à 5 autres hétéroatomes choisis parmi N, O, S, Si et P, ledit cycle étant éventuellement substitué par au moins un radical hydrocarboné ayant 1 à 20, notamment 1 à 15, et en particulier 1 à 6 atomes de carbone, linéaire, ramifié ou cyclique, saturé ou insaturé, comportant éventuellement 1 à 5 hétéroatomes choisis parmi N, O, S, Si et P;
- $R_4$ peut représenter un groupement hydrocarboné ayant 1 à 20, notamment 1 à 15, et en particulier 1 à 6 atomes de carbone, linéaire, ramifié ou cyclique, saturé ou insaturé, éventuellement halogéné ou perhalogéné, notamment par F, Br et/ou Cl, et/ou éventuellement comportant 1 à 5 hétéroatomes choisis parmi N, O, S, Si et P.

En l'occurrence $R_5$ et $R_6$ peuvent représenter, indépendamment l'un de l'autre, un groupement choisi parmi :

- les groupements aminoaryles cycliques saturés de formule (IIA) ou (IIB) :

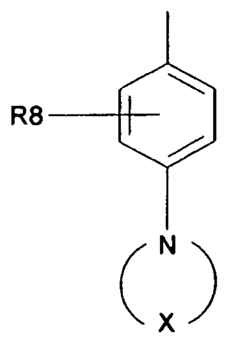

(IIA)

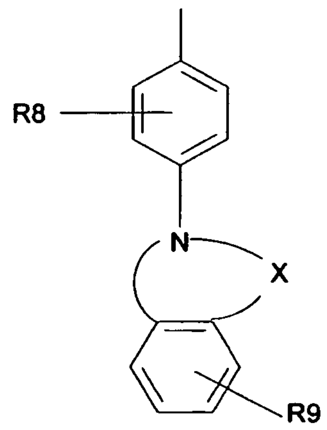

(IIB)

  dans lesquelles le cycle comportant N et X est un cycle saturé qui comprend au total 3 à 30 atomes, notamment 4 à 10 et encore en particulier 5, 6 ou 7 atomes, inclus l'azote, le reste étant des atomes de carbone et/ou des hétéroatomes choisis parmi O, S, Si, P et/ou des groupements choisis parmi -NH et -NR avec R représentant un radical hydrocarboné ayant 1 à 20, notamment 1 à 15, et en particulier 1 à 6 atomes de carbone, linéaire, ramifié ou cyclique, saturé ou insaturé, éventuellement comportant 1 à 5 hétéroatomes choisis parmi N, O, S, Si et P;
- un groupement hydrocarboné ayant 1 à 30, notamment 2 à 18, et en particulier 3 à 12 atomes de carbone, linéaire, ramifié ou cyclique, saturé ou insaturé, éventuellement comportant 1 à 5 hétéroatomes choisis parmi N, O, S, Si et P; et notamment un groupement choisi parmi $-C_6H_4-CONR_2R_3$, $-C_6H_4-NR_2R_3$ et $-C_6H_4-OR_4$ avec $R_2$, $R_3$ et $R_4$ ayant les significations données ci-dessus;

Notamment, $R_7$ peut représenter un groupement choisi parmi :

- (i) les groupements hydrocarbonés ayant 1 à 30, notamment 1 à 18, et en particulier 1 à 12 atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné, notamment par F, Br et/ou Cl;
- (ii) les atomes d'halogène, et notamment F, Br et/ou Cl;
- (iii) les groupements -CN (nitrile), -COOH (carboxylate), $-NO_2$ (nitro); - N=N- (azo); =NH (imino); $-CONH_2$ (amide);
- (iv) un atome d'hydrogène;
- (v) un groupement choisi parmi $-NR_2R_3$, $-OR_4$ ou $-SR_4$ avec $R_2$, $R_3$ et $R_4$ ayant les significations données ci-dessus;

- (vi) le radical $R_7$ pouvant en outre former, avec l'une des liaisons "i", "j", "k", ou "g,h" prises avec le radical $R_1$, ou "f" prise avec le radical $R_1$, un cycle hydrocarboné saturé ayant au total 3 à 8, notamment 4 à 7, et en particulier 5 ou 6 atomes de carbone, éventuellement comportant 1 à 5 hétéroatomes choisis parmi N, O, S, Si et P.

En particulier, $R'_1$ peut représenter l'hydrogène ou un groupement hydrocarboné ayant 1 à 30, notamment 1-18, et en particulier 1 à 12 atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné, notamment par F, Br et/ou Cl.

Notamment, $R'_2$ représente l'hydrogène ou un groupement choisi parmi $-NO_2$, - $NR_2R_3$ et $-C(O)NR_2R_3$, dans lesquels $R_2$ et $R_3$, soit représentent, indépendamment l'un de l'autre, un groupement hydrocarboné ayant 1 à 20, notamment 1 à 12, et en particulier 1 à 6, atomes de carbone, linéaire, ramifié ou cyclique, saturé ou insaturé, éventuellement comportant 1 à 5 hétéroatomes choisis parmi N, O, S, Si et P; soit pris ensemble avec l'atome d'azote auquel ils sont reliés, forment un hétérocycle hydrocarboné, saturé ou insaturé, comportant 3 à 10, notamment 4 à 6 atomes de carbone et éventuellement 1 à 5 autres hétéroatomes choisis parmi N, O, S, Si et P, ledit cycle étant éventuellement substitué par au moins un radical hydrocarboné ayant 1 à 20, notamment 1 à 15, et en particulier 1 à 6 atomes de carbone, linéaire, ramifié ou cyclique, saturé ou insaturé, comportant éventuellement 1 à 5 hétéroatomes choisis parmi N, O, S, Si et P.

On peut plus particulièrement citer les matières colorantes photochromes de formule (I) ou (Ia) pour lesquelles :

- $R_1$ représente l'hydrogène; ou un groupement -COOR avec R étant un radical hydrocarboné saturé ayant 1 à 12, notamment 1 à 6 atomes de carbone, et notamment un radical méthyle ou éthyle; ou un groupement

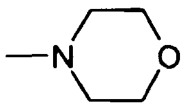

et/ou

- $R_5$ et $R_6$ représentent, indépendamment l'un de l'autre, soit (i) un groupement de formule (IIA) :

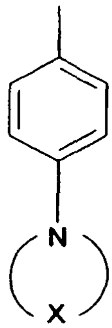

(IIA)

dans laquelle le cycle comportant N et X est un cycle saturé qui comprend au total 4 à 7 atomes, notamment 5 ou 6 atomes, inclus l'azote, et notamment 3 à 5 atomes de carbone et 0 ou 1 atome d'oxygène; et en particulier un groupement de formule:

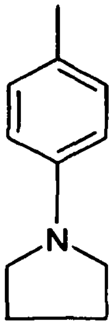
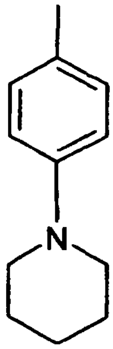
ou
ou
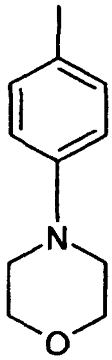

soit (ii) un groupement hydrocarboné ayant 5 à 14, notamment 6 à 10 atomes de carbone, linéaire, ramifié ou cyclique, saturé ou insaturé, éventuellement comportant 1 à 2 hétéroatomes choisis parmi N, O ou S;
en particulier un groupement

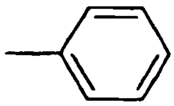
ou
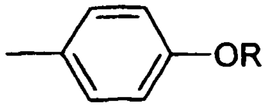
ou
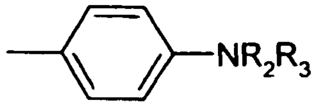

dans lesquelles R est un radical hydrocarboné saturé ayant 1 à 12, notamment 1 à 6 atomes de carbone, et notamment un radical méthyle ou éthyle; et $R_2$ et $R_3$ sont, indépendamment l'un de l'autre, un groupement hydrocarboné ayant 1 à 20, notamment 1 à 15, et en particulier 1 à 6 atomes de carbone, linéaire, ramifié ou cyclique, saturé ou insaturé, éventuellement comportant 1 à 5 hétéroatomes choisis parmi N, O, S, Si et P;
et/ou

- $R_7$ représente un atome d'hydrogène ou un groupement $-NR_2R_3$, avec $R_2$ et $R_3$ représentant, indépendamment l'un de l'autre, un groupement hydrocarboné saturé, linéaire ou ramifié, ayant 1 à 12, notamment 1 à 6 atomes de carbone, et notamment un groupement méthyle et/ou éthyle.

On peut également citer les colorants organiques de formule (II) ou (IIa) pour lesquelles :

- $R'_1$ représente l'hydrogène ou un groupement -COOR avec R étant un radical hydrocarboné saturé ayant 1 à 12, notamment 1 à 6 atomes de carbone, et notamment un radical méthyle ou éthyle;
et/ou
- $R_5$ et $R_6$ représentent, indépendamment l'un de l'autre, soit (i) un groupement de formule (IIA) :

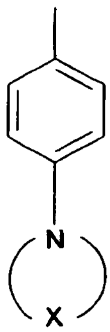
(IIA)

dans laquelle le cycle comportant N et X est un cycle saturé qui comprend au total 4 à 7 atomes, notamment 5 à 6 atomes, inclus l'azote, et notamment 4 à 5 atomes de carbone et 0 ou 1 atome d'oxygène; et en particulier un groupement de formule:

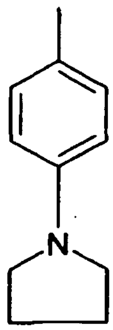

ou

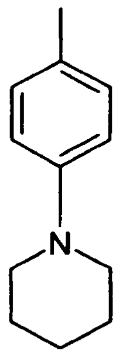

ou

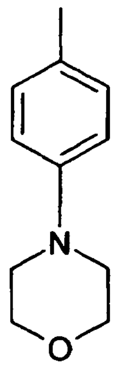

soit (ii) un groupement hydrocarboné ayant 5 à 14, notamment 6 à 10 atomes de carbone, linéaire, ramifié ou cyclique, saturé ou insaturé, éventuellement comportant 1 à 2 hétéroatomes choisis parmi N, O ou S;
en particulier un groupement :

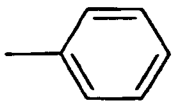

ou

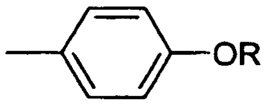

ou

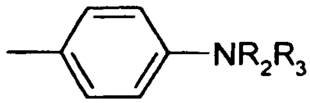

dans lesquelles R est un radical hydrocarboné saturé ayant 1 à 12, notamment 1-6 atomes de carbone, et notamment un radical méthyle ou éthyle; et $R_2$ et $R_3$ sont, indépendamment l'un de l'autre, un groupement hydrocarboné ayant 1 à 20, notamment 1 à 15, et en particulier 1 à 6 atomes de carbone, linéaire, ramifié ou cyclique, saturé ou insaturé, éventuellement comportant 1 à 5 hétéroatomes choisis parmi N, O, S, Si et P;
et/ou

- $R'_2$ représente l'hydrogène, ou un groupement -NR'R" avec R' et R", identiques ou différents, représentant un groupement hydrocarboné saturé, linéaire ou ramifié, ayant 1 à 12, notamment 1 à 6 atomes de carbone, et notamment un groupement méthyle et/ou éthyle; ou un groupement

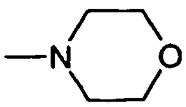

Conviennent tout particulièrement à l'invention, les dérivés tels que définis ci-dessus, dotés d'une activité photochrome.

A titre représentatif et non limitatif des matières colorantes de type naphtopyrane, on peut citer celles décrites dans les demandes WO94/22850, WO98/45281 et WO00/18755.

Plus précisément, conviennent tout particulièrement à l'invention les composés suivants :

Le 3,3-di(4-méthoxyphényl)-6-morpholino-3H-naphto[2,1-b]pyrane de formule :

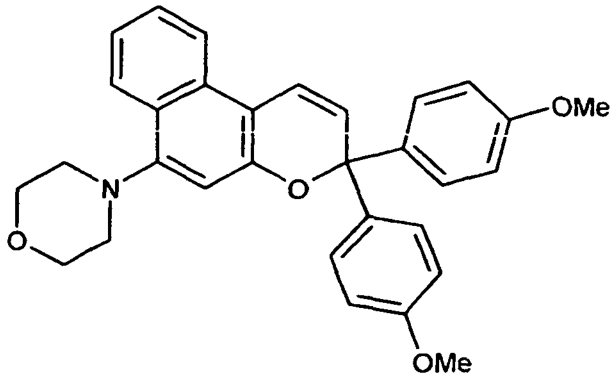

Le 3-phényl-3-(4-morpholinophényl)-6-morpholino-3H-naphto[2,1-b]pyrane de formule :

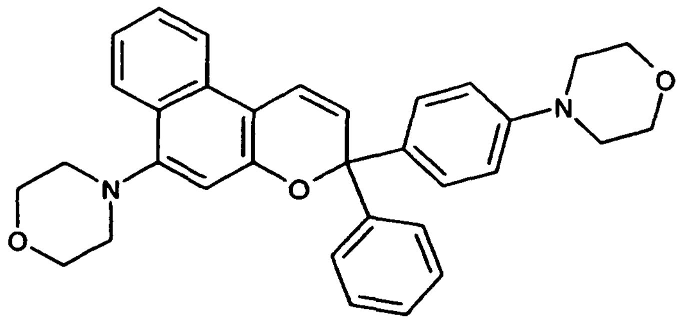

Le 3-phényl-3-(4-pipéridinophényl)-6-morpholino-3H-naphto[2,1-b]pyrane de formule :

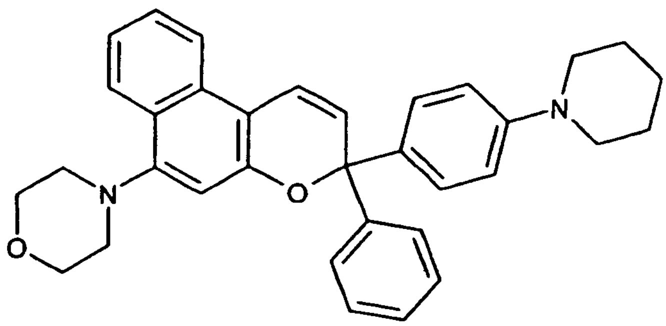

Le 3-phényl-3-(4-pipéridinophényl)-6-carboxyméthyl-9-N-diméthyl-3H-naphto[2,1-b]pyrane de formule :

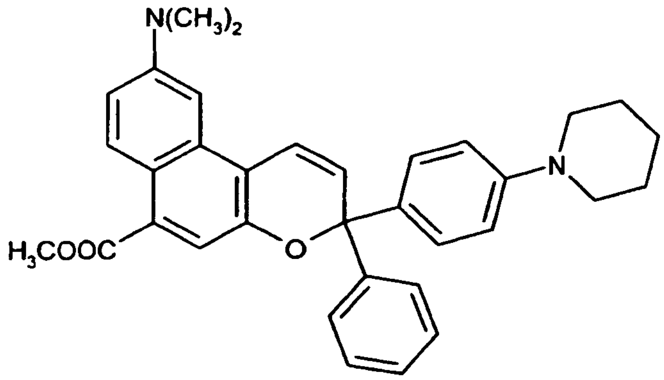

Le 2-phényl-2-(4-pipecidinophényl)-5-carboxyméthyl-9-N-diméthyl-2H-naphto[1,2-b]pyrane de formule :

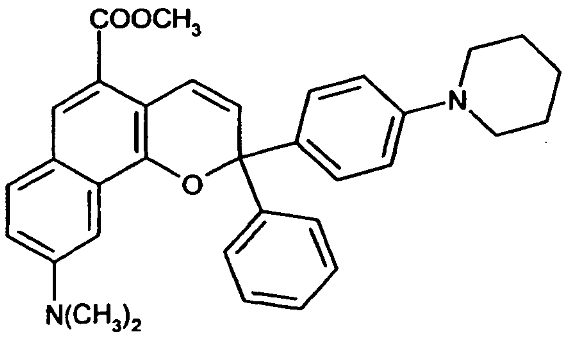

et leurs mélanges.

## AGENTS DE COLORATION GONIOCHROMATIQUES

Par « agent de coloration gionochromatique », on désigne au sens de la présente invention un agent de coloration permettant d'obtenir, lorsque la composition le contenant est étalée sur un support, un trajet de couleur dans le plan a*b* de l'espace colorimétrique CIE 1976 qui correspond à une variation Dh de l'angle de la teinte h d'au moins 20° lorsque l'on fait varier l'angle d'observation par rapport à la normale entre 0° et 80°, pour un angle d'incidence de la lumière de 45°.

Le trajet de couleur peut être mesuré par exemple au moyen d'un spectrogonioréflectomètre de marque INSTRUMENT SYSTEMS et de référence GON 360 GONIOMETER, après que la composition cosmétique ait été étalée à l'état fluide avec une épaisseur de 300 μm au moyen d'un étaleur automatique sur une carte de contraste de marque ERICHSEN et de référence Typ 24/5, la mesure étant effectuée sur le fond noir de la carte.

L'agent de coloration gioniochromatique peut être présent par exemple en une quantité pouvant varier, en poids par rapport au poids total de la composition le contenant, de 0,1 à 60 %, voire de 1 à 20 % ou de 2 à 15 %, et mieux de 2 à 10 %, notamment pour une composition destinée à être appliquée sur les lèvres. Une composition de vernis à ongles pourra contenir par exemple entre 0,1 % et 5 % d'agent de coloration goniochromatique ; un fond de teint pourra en contenir de 10 à 15 % et un rouge à lèvres pourra en contenir de 2 à 8 % en poids.

L'agent de coloration gionochromatique peut être choisi de manière à présenter un changement de couleur relativement important avec l'angle d'observation.

L'agent de coloration gionochromatique peut être choisi par exemple parmi les structures multicouche interférentielles et les agents de coloration à cristaux liquides.

Dans le cas d'une structure multicouche, celle-ci peut comporter par exemple au moins deux couches, chaque couche, indépendamment ou non de la (ou les) autre(s) couche(s), étant réalisée par exemple à partir d'au moins un matériau choisi dans le groupe constitué par les matériaux suivants : $MgF_2$, $CeF_3$, ZnS, ZnSe, Si, $SiO_2$, Ge, Te, $Fe_2O_3$, Pt, Va, $Al_2O_3$, MgO, $Y_2O_3$, $S_2O_3$, SiO, $HfO_2$, $ZrO_2$, $CeO_2$, $Nb_2O_5$, $Ta_2O_5$, $TiO_2$, Ag, Al, Au, Cu, Rb, Ti, Ta, W, Zn, $MoS_2$,

cryolithe, alliages, polymères et leurs associations.

Quant aux particules goniochromatiques à cristaux liquides susceptibles d'être mises en oeuvre dans la composition selon l'invention, elles peuvent notamment être à base de polymère susceptible d'être obtenu par polymérisation d'un mélange de monomères comprenant :

- a) au moins un premier monomère A de formule (I) Y1-A1-M1-A2-Y2 dans laquelle
  - i) Y1 et Y2 , identiques ou différents, représentent un groupe polymérisable choisi parmi les groupements acrylate ou méthacrylate, un groupement époxy, un groupe isocyanate, hydroxy, vinyléther ($-O-CH=CH_2$) ou vinylester ($-CO-O-CH=CH_2$), et
  - ii) A1 et A2, identiques ou différents, représentent un groupement de formule $-CnH_2n-$, dans laquelle n est un nombre entier allant de 0 à 20 et un ou plusieurs groupes méthylène dudit groupement $-CnH_2n-$ pouvant être remplacés par un ou plusieurs atomes d'oxygène, et
  - iii) M1 désigne un groupement de formule générale (I') $-R_1-X_1-R_2-X_2-R_3-X_3-R_4-$, dans laquelle $R_1$, $R_2$, $R_3$ et $R_4$, identiques ou différents, désignent un groupement divalent choisi parmi -O-, -COO-, -CONH-, -CO-, -S-, -C≡C-, -CH=CH-, -N=N-, -N=N(O)-, et $-R_2-X_2-R_3-$ ou $-R_2-X_2-$ ou $-R_2-X_2-R_3-X_3-$ pouvant également être une liaison covalente simple, et $X_1$,$X_2$,$X_3$ sont des groupements, identiques ou différents, choisis parmi les groupements 1,4-phénylène, 1,4-cyclohexylène, les groupements arylènes ou hétéroarylènes ayant un noyau aryle comprenant de 6 à 10 atomes éventuellement substitués par B1 et/ou B2 et/ou B3, ledit hétéroarylène contenant de 1 à 3 hétéroatomes choisis parmi les atomes O, N et S, les groupements cycloalkylènes ayant de 3 à 10 atomes de carbones éventuellement substitués par -B1 et/ou -B2 et/ou -B3, -B1, -B2, et -B3 , identiques ou différents, étant choisis parmi les groupes alkyle en $C_1$-$C_{20}$, alkoxy en $C_1$-$C_{20}$, alkylthio en $C_1$-$C_{20}$, alkyl($C_1$-$C_{20}$) carbonyl, alkoxy($C_1$-$C_{20}$) carbonyl, alkyl($C_1$-$C_{20}$) thiocarbonyl, -OH, -F, -Cl, -Br, -I, -CN, $-NO_2$, formyle, acétyle, et des groupements alkyle, alkoxy ou alkylthio ayant de 1 à 20 atomes de carbone, interrompus par un ou plusieurs atome(s) d'oxygène ou un ou plusieurs atome(s) de soufre ou un ou plusieurs groupe(s) ester,

  et
- b) au moins un deuxième monomère B chiral de formule (II) V1-A'1-W1-Z-W2-A'2-V2, dans laquelle
  - i) V 1 et V2 , identiques ou différents, désignent un groupement choisi parmi un groupement acrylate ou méthacrylate, un groupement époxy, un groupement vinyléther ou vinylester, un groupement isocyanate, un alkyle en $C_1$-$C_{20}$, un alkoxy en $C_1$-$C_{20}$, un alkylthio en $C_1$-$C_{20}$, un alkoxy($C_1$-$C_{20}$) carbonyl, un alkyl($C_1$-$C_{20}$)thiocarbonyle, -OH, -F, -Cl, -Br, -I, -CN, -NO2, formyle, acétyle et des groupements alkyle, alkoxy ou alkylthio, ayant de 1 à 20 atomes de carbone, et interrompus par un ou plusieurs atome(s) d'oxygène ou un ou plusieurs atome (s) de soufre ou un ou plusieurs groupe(s) ester (-CO-O-),
    et au moins V1 ou V2 désigne un groupe polymérisable choisi parmi les groupements acrylate ou méthacrylate, un groupement époxy, un groupe isocyanate, hydroxy, vinyléther ($-O-CH=CH_2$) ou vinylester ($-CO-O-CH=CH_2$),
  - ii) A'1 et A'2 , identiques ou différents, représentent un groupement de formule $-C_nH_{2n}-$, dans laquelle n est un nombre entier allant de 0 à 20 et un ou plusieurs groupes méthylène dudit groupement $C_nH_{2n}$ pouvant être remplacés par un ou plusieurs atomes d'oxygène, et
  - iii) W1 et W2 désignent un groupement divalent de formule générale $R'_1-X'_1-R'_2-X'_2-R'_3-$ dans laquelle $R'_1$, $R'_2$ et $R'_3$, identiques ou différents, désignent un groupement divalent choisi parmi -O-, -COO-, -CONH-, -CO-, -S-, -C≡C-, -CH=CH-, -N=N-, -N=N(O)-, et $R'_1$, $R'_2$, $R'_3$ ou $R'_2-X'_2$ peut également être une liaison covalente simple, et $X'_1$ et $X'_2$ sont des groupements, identiques ou différents, choisis parmi les groupements 1,4-phénylène, 1,4-cyclohexylène, les groupements arylènes ou hétéroarylènes ayant un noyau aryle comprenant de 6 à 10 atomes éventuellement substitués par B'1 et/ou B'2 et/ou B'3, ledit hétéroarylène contenant de 1 à 3 hétéroatomes choisis parmi les atomes O, N et S, les groupements cycloalkylènes ayant de 3 à 10 atomes de carbones éventuellement substitués par -B'1 et/ou -B'2 et/ou -B'3, -B'1, -B'2, et -B'3, identiques ou différents, étant choisis parmi les groupes alkyle en $C_1$-$C_{20}$, alkoxy en $C_1$-$C_{20}$, alkylthio en $C_1$-$C_{20}$, alkyl($C_1$-$C_{20}$)carbonyl, alkoxy ($C_1$-$C_{20}$) carbonyl, alkyl($C_1$-$C_{20}$)thiocarbonyl, -OH, -F, -Cl, -Br, -I, -CN, $-NO_2$, formyle, acétyle, et des groupements alkyle, alkoxy ou alkylthio ayant de 1 à 20 atomes de carbone, interrompus par un ou plusieurs atome(s) d'oxygène ou un ou plusieurs atome(s) de soufre ou un ou plusieurs groupe(s) ester,

et Z désigne un groupement chiral divalent comprenant au moins 4 atomes de carbone, notamment de 4 à 20 atomes de carbone, et mieux de 4 à 10 atomes de carbone, (le groupement chiral divalent comprenant au moins un carbone asymétrique, notamment un ou deux carbones asymétriques, et en particulier deux carbones asymétriques) et en particulier un groupe chiral divalent issu du groupe des dianhydrohexites, hexoses, pentoses, les dérivés binaphthyle (groupements binaphtyle), les dérivés biphényle (groupements biphényle), les dérivés d'acides tartriques ou des glycols

optiquement actifs.

De préférence, le polymère à cristaux liquides est obtenu par polymérisation d'un mélange de monomères comprenant :

- a) au moins un premier monomère A de formule (I) Y1-A1-M1-A2-Y2 dans laquelle

  -i) Y1 et Y2 , identiques ou différents, représentent un groupe acrylate ou méthacrylate, de préférence un groupe acrylate ;
  - ii) A1 et A2, identiques ou différents, représentent un groupement de formule -Cn$H_2$n-, dans laquelle n est un nombre entier allant de 1 à 20, de préférence allant de 2 à 6, et mieux égal à 4 ;
  - iii) M1 désigne un groupement de formule générale (I') $-R_1-X_1-R_2-X_2-R_3-X_3-R_4-$, dans laquelle R1 et R4 désignent -O-, $R_2$ et $R_3$ désignent -COO-,

  et X1,X2,X3 sont un groupement 1,4 phénylène, le groupe carbonyl -CO-respectivement de $R_2$ et de $R_3$ étant lié respectivement au groupement X1, X3,
  et
- b) au moins un deuxième monomère B chiral de formule (II) V1-W1-Z-W2-V2, dans laquelle

  - i) V1 désigne un groupement acrylate ou méthacrylate, et de préférence un groupe acrylate, et V2 désigne un groupement alkyle en $C_1$-$C_{20}$, un alkoxy en $C_1$-$C_{20}$, un alkoxy($C_1$-$C_{20}$)carbonyle, -OH, et de préférence désigne un groupement alkoxy en $C_1$-$C_{20}$, notamment en $C_1$-$C_4$, et en particulier un groupement méthoxy ;
  ii) W1 représente un groupement divalent de formule -X' 1-CO-O-,

  W2 représente un groupement divalent de formule -O-CO-X'1-,
  dans lesquelles X'1 désigne un groupe 1,4-phénylène,
  et Z désigne un groupement chiral à deux liaisons, issu du groupement dianhydrohexite, en particulier un radical divalent de formule :

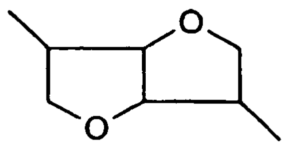

De préférence, le mélange de monomères comprend de 70 à 99 % en poids de monomère A et de 1 à 30 % en poids de monomère B, par rapport au poids total de monomère A et de monomère B, et mieux comprend de 90 à 95 % en poids de monomère A et de 5 à 10 % en poids de monomère B.

De préférence, la concentration des groupes polymérisables présents dans le mélange de monomère A et de monomère B (groupes polymérisables Y1, Y2 du monomère A et groupes polymérisables V1, V2 du monomère B) va de 3,2 à 15 mmoles/g.

Selon un mode particulier de réalisation de l'invention, le polymère à cristaux liquides est tel que le mélange de monomère A et de monomère B comprend des groupes polymérisables, dont au moins 90 % sont présents dans des monomères ayant au moins deux groupes polymérisables, en une concentration allant de 3,2 à 15 mmoles/g.

En particulier, le polymère à cristaux liquide comprend essentiellement ou consiste en un mélange des monomères A et B définis précédemment.

Le polymère à cristaux liquide présente notamment un pas d'hélice supérieur à 450 nm, notamment allant de 455 nm à 5000 nm, en particulier allant de 455 nm à 1000 nm, voire allant de 455 nm à 650 nm.

Le monomère A peut posséder un poids moléculaire moyen en poids allant de 150 à 800, et notamment allant de 460 à 625. Le monomère A peut être en particulier un dérivé de dibenzoate d'hydroquinone non substitué.

Le monomère B peut avoir un poids moléculaire moyen en poids allant de 500 à 1000, et notamment allant de 500 à 700.

Le polymère à cristaux liquides peut posséder un poids moléculaire moyen en poids inférieur à 625.

Le polymère à cristaux liquides défini précédemment peut être préparé suivant les procédés connus dans l'état de la technique, tels que ceux décrits dans les documents US 5362315, US 5807497, à partir du mélange de monomère décrit précédemment.

La polymérisation du mélange de monomères orienté peut, de manière déjà connue, se faire par exemple de façon radicalaire avec utilisation d'initiateurs thermiques du commerce, en utilisant des rayons d'électrons ou de la lumière UV en combinaison avec des photoinitiateurs du commerce, ou bien par des réactions d'addition ou de condensation.

La réticulation des mélanges de monomères, dans l'état structurel chiral, se fait de préférence au moyen d'une polyréaction qui, selon le type des groupes polymérisables, polycondensables ou polyadditionnables, se déroule sous la forme d'une polymérisation radicalaire, ionique ou catalysée au métal, ou d'une réaction de polycondensation ou d'une réaction de polyaddition.

L'initiation de la polymérisation radicalaire peut s'effectuer au moyen d'initiateurs correspondants ou par un rayonnement par UV, en utilisant des photoinitiateurs du commerce ou par un rayonnement à haute énergie, tel qu'un rayonnement d'électrons. Un avantage de la polymérisation thermique des radicaux ou de la polymérisation via un durcissement aux rayons d'électrons réside dans le fait qu'au mélange polymérisable peut également être ajouté un agent de protection contre la lumière, tel qu'un absorbeur d'UV (UVA) ou des capteurs de radicaux (HALS), pour stabiliser les pigments ou les films résultants face à la lumière UV, par exemple pour des applications extérieures, sans entraîner des pertes au niveau de la conversion de polymérisation, tel que ceci est le cas lors du durcissement aux UV, du fait de l'effet d'écrantage du photoinitiateur par un UVA. Il n'y a ainsi aucune diminution de la densité de réticulation.

Si le durcissement des films se fait de façon peroxydique ou par un rayonnement d'électrons, le mélange de monomères contient de préférence des agents de protection contre la lumière, du commerce, tels que des absorbeurs à UV ou des capteurs de radicaux, en une concentration globale de 0,5 à 5 % en poids.

Outre les photostabilisateurs, les mélanges de monomères peuvent également contenir d'autres additifs usuels visant à inhiber l'oxydation, à inhiber la polymérisation, ou des additifs visant à améliorer les propriétés rhéologiques. De plus, les charges absorbantes, telles que les pigments ou la suie, ainsi que des colorants ou des pigments à fluorescence peuvent être contenus.

Le film obtenu après la polymérisation est ensuite broyé en particules, notamment sous forme de plaquettes.

De préférence, les particules de polymère à cristaux liquides ont une plus grande taille allant de 1 μm à 3 mm, et de préférence allant de 30 μm à 500 μm. Ces particules sont avantageusement en forme de plaquettes.

Les particules peuvent être séparées (triées) par un procédé à sélectivité de la taille de grains.

De tels polymères et leurs particules sont décrits dans la demande EP-A-1046692.

Comme particules de polymère à cristaux liquides, on peut notamment utiliser celles connues sous le nom CTFA Polyacrylate-4 et vendus sous les dénominations « HELICONE® HC Sapphire », « HELICONE® HC Scarabeus », « HELICONE® HC Jade », « HELICONE® HC Maple », « HELICONE® HC XL Sapphire », « HELICONE® HC XL Scarabeus », « HELICONE® HC XL Jade », « HELICONE® HC XL Maple » par la société WACKER.

Les particules du polymère à cristaux liquide peuvent être présentes dans le produit selon l'invention à une teneur allant de 0,01 % à 99 % en poids, par rapport au poids total de la composition les contenant, notamment allant de 0,1 % à 60 % en poids, en particulier allant de 1 % à 30 % en poids, et voire allant de 5 % à 15 % en poids.

## AUTRES MATIÈRES COLORANTES

Au sens de la présente invention, on entend couvrir sous ce terme, les matières colorantes non goniochromatiques et/ou distinctes des matières colorantes dites premières matières colorantes selon l'invention. Il s'agit plus particulièrement de tout composé choisi parmi les colorants monochromes liposolubles et hydrosolubles, les nacres et les particules réfléchissantes.

### *Colorants monochromes liposolubles et hydrosolubles*

La première et/ou la seconde composition(s) peuvent en outre comprendre au moins un colorant monochrome, notamment un colorant organique naturel tel que le carmin de cochenille, et/ou un colorant de synthèse tel que les colorants halogéno-acides, azoïques, anthraquinoniques. On peut également citer des colorants minéraux tels que le sulfate de cuivre ou de fer. On peut encore citer le brun Soudan, le rouge Soudan et le rocou, ainsi que le jus de betterave, le carotène et le bleu de méthylène.

Le colorant peut être présent dans la composition considérée, seul ou en mélange, à raison de 0,001 à 15 % en poids, et notamment de 0,01 à 5 % en poids et notamment de 0,1 à 2 % en poids, par rapport au poids total de ladites composition.

### *Les pigments*

Par pigments, il faut comprendre des particules blanches ou colorées, minérales ou organiques, destinées à colorer et/ou opacifier la composition.

Des pigments peuvent être présents dans la première et/ou la seconde composition(s) notamment à raison de 0,01 à 25 % en poids de la composition considérée, et de préférence à raison de 3 à 10 % en poids.
Ils peuvent être blancs ou colorés, minéraux ou organiques, de taille usuelle ou nanométrique. Ils peuvent se présenter sous forme de poudre ou de pâte pigmentaire. On peut citer les oxydes de titane, de zirconium ou de cérium, ainsi que les oxydes de zinc, de fer ou de chrome, le bleu ferrique, l'hydrate de chrome, le noir de carbone, les outremers (polysulfures d'aluminosilicates), le pyrophosphate de manganèse et certaines poudres métalliques telles que celles d'argent ou d'aluminium. On peut encore citer les pigments D&C et les laques couramment employées pour conférer aux lèvres et à la peau un effet de maquillage, qui sont des sels de calcium, de baryum, d'aluminium, de strontium ou de zirconium.

***Les nacres***

Par nacres, il faut comprendre des particules irisées qui réfléchissent la lumière, notamment produites par certains mollusques dans leur coquille ou bien synthétisées.
Des nacres peuvent être présentes dans la première et/ou la seconde composition(s) notamment à raison de 0,01 à 20 % en poids, de préférence à un taux de l'ordre de 3 à 10 % en poids par rapport au poids total de la composition considérée. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.

***Les particules réfléchissantes***

Par « particules réfléchissantes », on désigne au sens de la présente invention des particules dont la taille, la structure, notamment l'épaisseur de la ou des couches qui la constituent et leur natures physique et chimique, et l'état de surface, leur permettent de réfléchir la lumière incidente avec une intensité suffisante pour pouvoir créer à la surface de la composition revendiquée, lorsque cette dernière est appliquée sur le support à maquiller, des points de surbrillance visibles à l'oeil nu, c'est-à-dire des points plus lumineux qui contrastent avec leur environnement en semblant briller.
Des particules réfléchissantes peuvent être présentes dans la première et/ou la seconde composition(s) en étant dispersées de manière homogène par exemple à une teneur allant de 0,1 % à 20 % par rapport au poids total de la composition, notamment de 1 % à 15 % en poids, et en particulier de 1 % à 10 % en poids, par exemple environ 2 % notamment pour une composition destinée à être appliquée sur les lèvres.
Les particules réfléchissantes qui permettent une réflexion métallique de la lumière incidente conviennent tout particulièrement. C'est le cas notamment lorsque les particules réfléchissantes permettent quelque soit leur forme, une réflexion sur une couche d'un métal, par exemple de l'argent. De telles particules s'avèrent relativement neutres vis-à-vis de la couleur de la composition.
Des particules réfléchissantes utilisables dans l'invention, à reflet métallique ou blanc, peuvent par exemple réfléchir la lumière dans toutes les composantes du visible sans absorber de manière significative une ou plusieurs longueurs d'ondes. La réflectance spectrale de ces particules réfléchissantes peut par exemple être supérieure à 70 % dans l'intervalle 400-700 nm, et en particulier d'au moins 80 %, voire 90 % ou encore 95 %.
La lumière réfléchie par les particules réfléchissantes peut être non iridescente, notamment dans le cas d'un reflet métallique.
Les particules réfléchissantes quelque soit leur forme, peuvent présenter une structure multicouche ou non et, dans le cas d'une structure multicouche, par exemple au moins une couche d'épaisseur uniforme, notamment d'un matériau réfléchissant.
Le matériau réfléchissant peut comporter une couche de métal ou d'un composé métallique.
Des particules de verre recouvertes d'une couche métallique sont décrites notamment dans les documents JP-A-09188830, JP-A-10158450, JP-A-10158541, JP-A-07258460, JP-A-05017710.
La première et/ou la seconde composition(s) peuvent en outre comprendre au moins un composé photochrome mais distinct de ceux considérés selon l'invention c'est-à-dire caractérisé d'une manière générale par un $\Delta E < 5$ et le cas échéant une insolubilité dans la composition considérée. On peut notamment citer les composés photochromes minéraux, et plus particulièrement les aluminosilicates dopés tels que la sodalite dopée par des halogènes, ou les oxydes ou hydrates métalliques tels que les oxydes de titane rendu photochrome à l'aide d'un métal choisi parmi le fer, le chrome, le cuivre, le nickel, le manganèse, le cobalt, le molybdène, tel quel ou sous forme de sel tel qu'un sulfate, un chlorate, un nitrate, un acétate. Un tel composé photochrome peut être incorporé dans la première composition et/ou la seconde composition(s) en une quantité de 0,001 à 20 % poids par rapport au poids total de la composition considérée, notamment en une quantité de 0,1 à 10 % en poids.

**MILIEU PHYSIOLOGIQUEMENT ACCEPTABLE**

Les milieux physiologiquement acceptables de chacune des compositions composant le produit selon l'invention seront adaptés à la nature du maquillage à appliquer, la nature de la surface à maquiller ou à traiter sur laquelle doit être appliquée le produit ainsi qu'à la forme sous laquelle le produit est destinée à être conditionné, notamment solide ou fluide à température ambiante et pression atmosphérique.

La première et/ou la seconde composition(s) du produit selon l'invention peuvent comprendre au moins une phase aqueuse et/ou au moins une phase grasse.

La première et/ou la seconde composition(s) du produit selon l'invention peuvent être anhydre, ou également comprendre une phase aqueuse, qui peut comprendre de l'eau, une eau florale telle que de l'eau de bleuet et/ou une eau minérale telle que l'eau de VITTEL, l'eau de LUCAS ou l'eau de LA ROCHE POSAY.

Ladite phase aqueuse peut comprendre de 0,1 à 14 % en poids, par rapport au poids total de la phase aqueuse, d'un monoalcool en $C_2$-$C_6$ comme l'éthanol, le propanol, le butanol, l'isopropanol et l'isobutanol.

**Huiles**

Généralement, la première et/ou la seconde composition(s) du produit selon l'invention comprennent au moins une phase huileuse qui peut comprendre une ou plusieurs huiles cosmétiquement acceptables.

Par huile cosmétiquement acceptable selon l'invention, on entend tout corps gras liquide à 25°C, 1 atm, et ayant un poids moléculaire supérieur ou égal à 160, notamment compris entre 170 et 106, voire entre 200 et 5.105, compatible avec une application sur la peau, les muqueuses (lèvres) et/ou les phanères (ongles, cils, sourcils, cheveux).

De préférence, la phase huileuse est macroscopiquement homogène, c'est-à-dire homogène à l'oeil nu.

La phase huileuse peut comprendre une ou plusieurs huiles, qui peuvent être polaires ou apolaires, volatiles ou non volatiles, et de préférence, hydrocarbonées.

On entend par huile polaire, une huile composée de composés chimiques comportant au moins un groupement polaire. Les groupements polaires sont bien connus de l'homme du métier : il peut s'agir notamment de groupement de type alcool, ester ou acide carboxylique.

En particulier, on peut définir les huiles polaires selon l'invention comme ayant un paramètre moyen de solubilité $\delta a$ selon l'espace de solubilité de Hansen, à 25°C, de : $\delta a \geq 5{,}0\ (J/cm^3)^{½}$.

Les huiles polaires comprennent les huiles plutôt polaires qui ont un paramètre moyen de solubilité à 25°C de : $5{,}0 \leq \delta a \leq 7{,}0\ (J/cm^3)^{½}$, et les huiles franchement polaires qui ont un paramètre moyen de solubilité à 25°C de : $\delta a > 7{,}0\ (J/cm^3)^{½}$.

De la même manière, les huiles apolaires au sens de l'invention ont un paramètre moyen de solubilité $\delta a$ selon l'espace de solubilité de Hansen, à 25 °C, de :
$0 \leq \delta a < 5{,}0\ (J/cm^3)^{½}$.

Les huiles apolaires au sens de l'invention comprennent les huiles franchement apolaires ($\delta a = 0$) et les huiles peu polaires qui ont un paramètre moyen de solubilité à 25°C de : $0 < \delta a < 5{,}0\ (J/cm^3)^{½}$.

Ainsi, plus la valeur de $\delta a$ est élevée, plus la polarité de l'huile est élevée.

La définition et le calcul des paramètres de solubilité dans l'espace de solubilité tridimensionnel de HANSEN sont décrits dans l'article de C. M. HANSEN : "The three dimensionnal solubility parameters" J. Paint Technol. 39, 105 (1967).

Selon cet espace de Hansen :

- $\delta D$ caractérise les forces de dispersion de LONDON issues de la formation de dipôles induits lors des chocs moléculaires ;
- $\delta p$ caractérise les forces d'interactions de DEBYE entre dipôles permanents ainsi que les forces d'interactions de KEESOM entre dipôles induits et dipôles permanents;
- $\delta h$ caractérise les forces d'interactions spécifiques (type liaisons hydrogène, acide/base, donneur/accepteur, etc.) ;
- $\delta a$ est déterminé par l'équation : $\delta a = (\delta p^2 + \delta h^2)^{½}$

Les paramètres $\delta p$, $\delta h$, $\delta D$ et $\delta a$ sont exprimés en (J/cm3)½.

Lorsque la phase huileuse est un mélange de différentes huiles, les paramètres de solubilité du mélange sont déterminés à partir de ceux des composés pris séparément, selon les relations suivantes :

$$\delta_{Dmel} = \sum_i xi\ \delta Di \quad ; \quad \delta = \sum_i xi\ \delta pi \quad \text{et} \quad \delta = \sum_i xi\ \delta hi$$

$$\delta_{amel} = (\delta^2_{pmel} + \delta^2_{hmel})1/2$$

où xi représente la fraction volumique du composé i dans le mélange.

Il est à la portée de l'homme du métier de déterminer les quantités de chaque huile pour obtenir une phase huileuse satisfaisant aux critères souhaités.

Selon un premier mode de réalisation de l'invention, la phase huileuse est polaire et peut comprendre principalement, voire exclusivement, une ou plusieurs huiles polaires (plutôt ou franchement polaires) en mélange, qui peuvent donc représenter 5 à 100 % en poids, notamment de 10 à 90 %, voire de 15 à 60 % et en particulier de 20 à 50 % en poids du poids total de ladite phase huileuse.

Selon ce premier mode, la phase huileuse polaire présente un paramètre moyen de solubilité δa selon l'espace de solubilité de Hansen, à 25 °C, supérieur ou égal à 5,0 $(J/cm^3)^{½}$, notamment supérieur ou égal à 5,3, voire supérieur ou égal à 5,5 et encore mieux supérieur ou égal à 6,0 $(J/cm^3)^{½}$, voire supérieur ou égale à 7,0 $(J/cm^3)^{½}$.

Selon un second mode de réalisation de l'invention, la phase huileuse est apolaire et peut comprendre de 5 à 100 % en poids, notamment de 10 à 90 %, voire de 15 à 60 % et en particulier de 20 à 50 % en poids d'une ou plusieurs huiles apolaires (apolaires ou peu polaires); elle présente un paramètre moyen de solubilité δa selon l'espace de solubilité de Hansen, à 25 °C, inférieur à 5,0, notamment inférieur ou égal à 4,9, encore mieux inférieur ou égal à 4,5 et encore mieux inférieur ou égal à 4,0 $(J/cm^3)^{½}$.

Les huiles susceptibles d'être employées dans la phase huileuse peuvent être choisies parmi, seule ou en mélange, les huiles d'origine animale, végétale, minérale ou synthétique; volatiles ou non.

On entend par huile volatile, les huiles ayant, à 25 °C, une pression de vapeur comprise entre 0,02 et 300 mm Hg (soir 2,66 à 40000 Pa). De préférence, on utilise des huiles volatiles dont le point éclair est de l'ordre de 30-100°C.

On peut notamment citer :

- les huiles animales ou végétales notamment formées par des esters d'acide gras et de polyols, en particulier les triglycérides liquides, par exemple les huiles de tournesol, de maïs, de soja, de courge, de pépins de raisin, de sésame, de noisette, d'abricot, d'amandes ou d'avocat; les huiles de poisson, le tricaprocaprylate de glycérol, ou les huiles végétales ou animales de formule $R_1COOR_2$ dans laquelle $R_1$ représente le reste d'un acide gras supérieur comportant de 7 à 19 atomes de carbone et $R_2$ représente une chaîne hydrocarbonée ramifiée contenant de 3 à 20 atomes de carbone, par exemple, l'huile de Purcellin; l'huile de paraffine, de vaseline, de calophyllum, de macadamia, de colza, de coprah, d'arachide, de palme, de ricin, de jojoba, d'olive ou de germes de céréales; l'huile de beurre de karité; le perhydrosqualène;
- les esters et les éthers de synthèse notamment d'acides gras comme par exemple les huiles de formule $R_1COOR_2$ dans laquelle $R_1$ représente le reste d'un acide gras supérieur comportant de 7 à 29 atomes de carbone et $R_2$ représente une chaîne hydrocarbonée contenant de 3 à 30 atomes de carbone comme par exemple le myristate d'isopropyle, le palmitate d'éthyl-2-hexyle, le stéarate d'octyl-2-dodécyle, l'érucate d'octyl-2-dodécyle, l'isononanoate d'isononyle, l'isostéarate d'isostéaryle; les esters hydroxylés comme le lactate d'isostéaryle, l'hydroxystéarate d'octyle, l'hydroxystéarate d'octyldodécyle, le malate de diisostéaryle, le citrate de triisocétyle, des heptanoates, octanoates, décanoates d'alcools gras; des esters de polyol comme le dioctanoate de propylène glycol, le diheptanoate de néopentylglycol, le diisononanoate de diéthylèneglycol; les esters du pentaérythritol comme le tétraisostéarate de pentaérythrithyle ; des esters du type trimellitate de tridécyle ;
- des alcools gras notamment ayant de 12 à 26 atomes de carbone comme l'octyldodécanol, le 2-butyloctanol, le 2-hexyldécanol, le 2-undécylpentadécanol, l'alcool oléique ;
- des hydrocarbures linéaires ou ramifiés, d'origine minérale ou synthétique tels que les huiles de paraffine et leurs dérivés, la vaseline, les polydécènes, le polyisobutène hydrogéné tel que le parléam; les isoparaffines comme l'isohexadécane et l'isodécane ;
- des glycérides et notamment des acétylglycérides ou des triglycérides d'acides gras ayant 4 à 10 atomes de carbone, comme les triglycérides des acides heptanoïque, octanoïque et des acides caprique/caprylique,
- et leurs mélanges.

Parmi les huiles polaires particulièrement préférées, on peut citer l'octyldodécanol, l'hexyldécanol, l'octyldécanol, l'alcool oléique, l'huile de ricin, le malate de diisostéaryle, le triheptanoate de glycéryle, le trioctanoate de glycéryle, le triglycéride des acides caprique/caprylique, le triisononanoïn, le trimellitate de tridécyle, et leurs mélanges.

Parmi les huiles apolaires particulièrement préférées, on peut citer les hydrocarbures aliphatiques notamment en $C_6$-$C_{40}$ comme les huiles de paraffine, volatiles, telles que l'isohexadecane ou l'isododécane, ou non volatiles, et leurs dérivés; la vaseline, les polydécènes hydrogénés ou non, le polyisobutène hydrogéné tel que l'huile de Parléam, le squalane, les polybutylènes, l'isononanoate d'isononyle; les huiles fluorées notamment perfluorées, et leurs mélanges.

On peut notamment citer les huiles suivantes :

| | δa $(J/cm^3)^{½}$ |
|---|---|
| huile de ricin | 9,09 |
| 2-hexyldécanol | 8,55 |
| alcool oléïque | 8,17 |
| octyldodécanol | 7,69 |
| triglycéride de l'acide heptanoïque | 7,29 |
| diisostéarylmalate | 7,19 |
| triglycéride de l'acide octanoïque | 6,87 |
| triglycéride des acides caprique/caprylique | 6,69 |
| Triisononanoïne | 6,54 |
| Trimellilate de tridécyle | 5,35 |
| Isononanoate d'isononyle | 4,87 |
| Polyisobutène hydrogéné | 0 |
| Isododécane | 0 |

On choisit de préférence la phase huileuse de manière à ce que la ou les matières colorantes photochromes y soient à défaut de solubles, dispersibles en tant que telle c'est-à-dire sans traitement de surface annexe.

La phase huileuse est de préférence comprise en une quantité de 1 à 99 % en poids, notamment 10 à 90 % en poids, de préférence 15 à 80 % en poids, par rapport au poids total de la composition considérée.

La première et/ou la seconde composition(s) du produit selon l'invention peuvent comprendre en outre d'autres corps gras que les huiles ci-dessus, qui peuvent être choisis par l'homme du métier sur base de ses connaissances générales, de manière à conférer au produit final les propriétés souhaitées, par exemple en consistance et/ou en texture.

Ces corps gras additionnels peuvent être des cires, des gommes et/ou des corps gras pâteux d'origine animale, végétale, minérale ou synthétique, ainsi que leurs mélanges.

Une cire, au sens de la présente invention, est un composé gras lipophile, solide à température ambiante (25°C), à changement d'état solide/liquide réversible, ayant une température de fusion supérieure à 40°C et pouvant aller jusqu'à 200°C, généralement une dureté supérieure à 0,5 MPa, et présentant à l'état solide une organisation cristalline anisotrope.

On peut notamment citer les cires d'origine animale, végétale, minérale ou synthétique telles que les cires microcristallines, la cire de paraffine, le pétrolatum, la vaseline, l'ozokérite, la cire de montan; la cire d'abeilles, la cire de lanoline et ses dérivés; les cires de Candellila, d'Ouricury, de Carnauba, du Japon, le beurre de cacao, les cires de fibres de lièges ou de canne à sucre, la cire de lignite, la cire de son de riz, la cire de sapin, la cire de coton; les huiles hydrogénées ayant une température de fusion supérieure à 40°C (environ), comme l'huile de jojoba hydrogénée; les esters gras et les glycérides concrets à 25°C; les cires de polyéthylène et les cires obtenues par synthèse de Fischer-Tropsch; des huiles hydrogénées concrètes à 25°C; des lanolines.

Les corps gras pâteux ont généralement un point de fusion compris entre 25 et 60°C, de préférence entre 30 et 45°C, et/ou une dureté allant de 0,001 et 0,5 MPa, de préférence entre 0,005 et 0,4 MPa. On peut notamment citer les lanolines et leurs dérivés, ou les esters de cholestérol.

Ces corps gras additionnels peuvent être présents en une quantité de 0,1 à 50 % en poids, notamment de 3 à 40 % en poids, encore mieux de 5 à 30 % en poids, par rapport au poids total de la composition considérée.

## Tensioactifs

La première et/ou la seconde composition(s) du produit selon l'invention peuvent éventuellement comprendre un tensioactif, notamment lorsqu'elles se présentent sous forme d'émulsion, en particulier en une quantité de 0,01 à 30 % en poids par rapport au poids total de la composition le ou les contenant.

On peut citer, seuls ou en mélange, les sels alcalins, les sels d'ammonium, les sels d'amines ou les sels d'aminoalcools des composés suivants : les alcoylsulfates, alcoyléther sulfates, alcoylamides sulfates et éthers sulfates, alcoylarylpolyéthersulfates, monoglycérides sulfates, alcoylsulfonates, alcoylamides sulfonates, alcoylarylsulfonates, α-oléfines sulfonates, paraffines sulfonates, alcoylsulfosuccinates, alcoyléthersulfosuccinates, alcoylamides sulfosuccinates, alcoylsulfosuccinamates, alcoylsulfoacétates, alcoylpolyglycérol carboxylates, alcoylphosphates/alcoylétherphosphates, acylsarcosinates, alcoylpolypeptidates, alcoylamidopolypeptidates, acyliséthionates, alcoyllaurates. Le radical alcoyle ou acyle dans tous ces composés désigne généralement une chaîne de 12 à 18 atomes de carbone.

On peut aussi citer les savons et les sels d'acides gras tels que les acides oléique, ricinoléique, palmitique, stéarique, les acides d'huile de coprah ou d'huile de coprah hydrogénée et notamment les sels d'amines tels que les stéarates d'amines; les acyl lactylates dont le radical acyle comprend 8-20 atomes de carbone; les acides carboxyliques d'éthers polyglycoliques.

On peut encore citer les alcools, les alcoylphénols et acides gras polyéthoxylés, polypropoxylés ou polyglycérolés à chaîne grasse comportant 8 à 18 atomes de carbone; des copolymères d'oxydes d'éthylène et de propylène, des condensats d'oxyde d'éthylène et de propylène sur des alcools gras, des amides gras polyéthoxylés, des amines grasses polyéthoxylées, des éthanolamides, des esters d'acides gras de glycol, des esters d'acides gras (stéarate, oléate) du sorbitan oxyéthylénés ou non, des esters d'acides gras du saccharose, des esters d'acides gras de polyéthylèneglycol (monostéarate ou monolaurate de polyéthylène glycol); des triesters phosphoriques, des esters d'acides gras de dérivés de glucose; les alkylpolyglycosides et les alkylamides des sucres aminés; les produits de condensation d'un $\alpha$-diol, d'un monoalcool, d'un alcoylphénol, d'un amide ou d'un diglycolamide avec le glycidol ou un précurseur de glycidol.

On peut également citer le trioléyl phosphate; les ester d'acides gras de polyols comme les mono-, di-, tri- ou sesqui-oléates ou stéarates de sorbitol ou de glycérol, les laurates de glycérol ou de polyéthylène glycol; les alkyl ou alkoxy diméthicones copolyols à chaîne alkyle ou alkoxy pendante ou en bout de squelette siliconé ayant par exemple de 6 à 22 atomes de carbone; les alkyl (lauryl, cétyl, stéaryl, octyl) éthers polyoxyéthylénés et les diméthicones copolyols.

## Agent épaississant

La première et/ou la seconde composition(s) du produit selon l'invention peuvent encore comprendre un ou plusieurs agents épaississants, par exemple dans des concentrations de 0,01 à 6 % en poids par rapport au poids total de la composition le ou les contenant.

L'agent épaississant peut être choisi parmi, seul ou en mélange :

- les biopolymères polysaccharidiques comme la gomme de xanthane, la gomme de caroube, la gomme de guar, les alginates, les celluloses modifiées telles que l'hydroxyéthylcellulose, la méthylcellulose, l'hydroxypropylcellulose et la carboxyméthylcellulose, les dérivés de l'amidon, les dérivés d'éthers de cellulose possédant des groupes ammonium quaternaires, les polysaccharides cationiques;
- les polymères synthétiques comme les acides polyacryliques tels que les polymères poly(méth)acrylates de glycéryle tels que l'HISPAGEL ou le LUBRAGEL des sociétés HISPANO QUIMICA ou GARDIAN, la polyvinylpyrrolidone, l'alcool polyvinylique, les polymères réticulés d'acrylamide et d'acrylate d'ammonium tels que le PAS 5161 ou BOZEPOL C de HOECHST; les copolymères acrylate/octyl-acrylamide tels que le Dermacryl de National Starch; les polymères à base de polyacrylamide tels que le SEPIGEL 305 de SEPPIC, les polymères réticulés d'acrylamide et de chlorure de méthacryloyloxyéthyltriméthylammonium tels que le SALCARE SC 92 de ALLIED COLLOIDS,
- le silicate de magnésium et d'aluminium;
- les argiles éventuellement modifiées comme les hectorites modifiées par un chlorure d'ammonium d'acide gras en $C_{10}$ à $C_{22}$, comme l'hectorite modifiée par du chlorure de di-stéaryl di-méthyl ammonium ;
- la silice éventuellement modifiée;
- les galactomannanes comportant un à six et mieux de deux à quatre groupes hydroxyle par ose, substitués par une chaîne alkyle saturée ou non, comme la gomme de guar alkylée par des chaînes alkyle en $C_1$ à $C_6$ et mieux en $C_1$ à $C_3$ et plus particulièrement la guar éthylée ayant un degré de substitution de 2 à 3 telle que celle vendue par la société Aqualon sous le nom N-HANCE-AG ;
- les dérivés de cellulose tel que l'éthylcellulose.
- les copolymères séquencés, notamment de type "dibloc" ou "tribloc" du type polystyrène/polyisoprène, polystyrène/polybutadiène, polystyrène/copoly(éthylène-butylène) ou encore polystyrène/copoly(éthylène-propylène) tels que ceux vendus sous le nom de 'Kraton' par Shell Chemical;
- les polymères de type polyamide, par exemple comportant un squelette polymérique ayant des motifs répétitifs amide, et éventuellement au moins une chaîne grasse pendante et/ou au moins une chaîne terminale éventuellement fonctionnalisées, ayant de 8 à 120 atomes de carbone et étant liées à ces motifs amide, parmi lesquels on peut citer les produits commerciaux vendus par la société Arizona Chemical sous les noms Uniclear 80 et Uniclear 100 qui sont un mélange de copolymères d'un diacide en C36 condensé sur l'éthylène diamine, de masse moléculaire moyenne en poids d'environ 6000, les groupes ester terminaux résultant de l'estérification des terminaisons d'acide restantes par l'alcool cétylique, stéarylique ou leurs mélanges (appelés aussi alcool cétylstéarylique).

**Polymère filmogène**

Selon l'application envisagée, la première et/ou la seconde composition(s) du produit selon l'invention peuvent comprendre en outre au moins un polymère filmogène. Ceci est généralement le cas lorsque l'on souhaite préparer un produit de type vernis à ongles ou laque à lèvres.
Parmi les polymères filmogènes utilisables dans le produit de la présente invention, on peut citer les polymères synthétiques, de type radicalaire ou de type polycondensat, les polymères d'origine naturelle, et leurs mélanges.
Les polymères filmogènes de type radicalaire peuvent être notamment des polymères ou des copolymères vinyliques, notamment des polymères acryliques.
Les polymères filmogènes vinyliques peuvent résulter de la polymérisation de monomères à insaturation éthylénique ayant au moins un groupement acide et/ou des esters de ces monomères acides et/ou des amides de ces monomères acides comme les acides carboxyliques insaturés $\alpha,\beta$-éthyléniques tels que l'acide acrylique, l'acide méthacrylique, l'acide crotonique, l'acide maléique, l'acide itaconique.
Les polymères filmogènes vinyliques peuvent également résulter de l'homopolymérisation ou de la copolymérisation de monomères choisis parmi les esters vinyliques comme l'acétate de vinyle, le néodécanoate de vinyle, le pivalate de vinyle, le benzoate de vinyle et le t-butyl benzoate de vinyle et les monomères styrèniques comme le styrène et l'alpha-méthyl styrène. En particulier, ces monomères peuvent être polymérisés avec des monomères acides et/ou leurs esters et/ou leurs amides, tels que ceux mentionnés précédemment.
Parmi les polycondensats filmogènes, on peut citer les polyuréthanes, les polyesters, les polyesters amides, les polyamides, et les résines époxyesters, les polyurées.
Les polymères d'origine naturelle, éventuellement modifiés, peuvent être choisis parmi la résine shellac, la gomme de sandaraque, les dammars, les élémis, les copals, les polymères cellulosiques insolubles dans l'eau tels que la nitrocellulose, l'acétate de cellulose, l'acétobutyrate de cellulose, l'acétopropionate de cellulose, l'éthyl cellulose, et leurs mélanges.
Les polymères peuvent être dissous ou dispersés dans le milieu cosmétiquement acceptable de la première et/ou la seconde composition. Les polymères peuvent être présents à une teneur allant de 0,01 % à 40 % en poids par rapport au poids total de la composition le ou les contenant.
La première et/ou la seconde composition(s) du produit selon l'invention peuvent également comprendre un plastifiant choisi parmi les plastifiants usuels et qui peut être présent à une teneur allant de 0,1 à 40 % en poids par rapport au poids total de la composition le contenant.

**Charge**

La première et/ou la seconde composition(s) du produit selon l'invention peuvent comprendre en outre des charges habituellement utilisées dans les compositions cosmétiques.
Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage.
Les charges, qui peuvent être présentes à raison de 0,01 à 60 % en poids, de préférence de 3 à 10 %, dans la première et/ou la seconde composition du produit selon l'invention peuvent être minérales ou de synthèse, lamellaires ou non lamellaires. On peut citer le talc, le mica, la silice, le kaolin, les poudres de Nylon et de polyéthylène, le Téflon, l'amidon, le nitrure de bore, les microsphères de polymère telles que l'Expancel (Nobel Industrie), le polytrap (Dow Corning) et les microbilles de résine de silicone (Tospearls de Toshiba, par exemple), le carbonate de calcium précipité, le carbonate ou l'hydrocarbonate de magnésium, des savons métalliques dérivés d'acides organiques carboxyliques ayant de 8 à 22 atomes de carbone.

**Additifs**

Les première et seconde composition(s) du produit de maquillage selon l'invention peuvent également contenir un ou plusieurs actifs cosmétiques, dermatologiques, hygiéniques ou pharmaceutiques.
Comme actifs cosmétiques, dermatologiques, hygiéniques ou pharmaceutiques, utilisables dans les compositions de l'invention, on peut citer les hydratants (polyol comme glycérine), vitamines (C, A, E, F, B ou PP), acides gras essentiels, huiles essentielles, céramides, sphingolipides, filtres solaires liposolubles ou sous forme de nano-particules, les actifs spécifiques de traitement de la peau (agents de protection, anti-bactériens, antirides, ...).
Le produit selon l'invention peut également être exempt de filtre UV.
Ces actifs sont utilisés en quantité habituelle pour l'homme de l'art et notamment à des concentrations de 0 à 20 % et notamment de 0,001 à 15 % du poids total de la première ou seconde composition.
Chaque composition du produit selon l'invention peut, de plus, comprendre, selon le type d'application envisagée, les constituants classiquement utilisés dans les domaines considérés, qui sont présents en une quantité appropriée à la forme galénique souhaitée.

Chaque composition du produit selon l'invention peut se présenter sous toute forme galénique normalement utilisée pour une application topique et notamment sous forme d'une solution huileuse ou aqueuse, d'un gel huileux ou aqueux, d'une émulsion huile-dans-eau ou eau-dans-huile, d'une émulsion multiple, d'une dispersion d'huile dans de l'eau grâce à des vésicules, les vésicules étant situés à l'interface huile/eau, ou d'une poudre. Chaque composition peut être fluide ou solide.

Les compositions du produit selon l'invention peuvent être préparées de manière usuelle par l'homme du métier.

Elles peuvent se présenter sous forme d'un produit coulé et par exemple sous la forme d'un stick ou bâton, sous forme de pâte souple dans une bouillotte, ou sous la forme de coupelle utilisable par contact direct ou à l'éponge. En particulier, elle constituent, ensemble ou séparément, un fond de teint coulé, fard à joues ou à paupières coulé notamment coloré, rouge à lèvres, brillant pour les lèvres, produit anti-cernes ou contour des yeux.

Elles peuvent aussi se présenter chacune sous forme d'une pâte souple d'un onguent, d'une pommade, d'une lotion ou encore de gel, de crème plus ou moins fluide. Elle peuvent alors constituer des fonds de teint ou des rouges à lèvres fluides ou pâteux, des brillants à lèvres, des produits solaires ou de coloration de la peau, des eye-liners, des produits de maquillage du corps ou encore présenter des propriétés de soin et se présenter alors sous la forme de base ou de baume de soin des lèvres.

Avantageusement, la première ou la seconde composition, ou les deux, sont à phase graisseuse continue et de préférence sous forme anhydre et peuvent contenir moins de 5 % d'eau, et encore mieux moins de 1 % d'eau par rapport au poids total de chacune des compositions. En particulier, le produit de maquillage bicouche entier est sous forme anhydre.

De préférence, chaque composition se présente sous la forme d'un stick plus ou moins rigide.

Chaque composition peut être conditionnée séparément dans un même article de conditionnement comme par exemple dans un stylo bi-compartimenté.

De préférence, la composition qui est appliquée en première couche est sous forme solide, ce qui permet une application plus pratique, une meilleure stabilité dans le temps et en température de la composition et permet un tracé précis du maquillage, ce qui est hautement souhaitable dans le cas d'un rouge à lèvres ou d'un eye-liner.

Le produit selon l'invention peut être avantageusement utilisé pour le maquillage de la peau, des lèvres et/ou des phanères selon la nature des ingrédients utilisés.

De préférence, le produit selon l'invention est un produit de maquillage de la peau ou des lèvres. Plus particulièrement, il se présente sous la forme d'un rouge à lèvres bi-couches.

## Exemples

Les pourcentages ci-après sont tous exprimés en poids par rapport au poids total de la composition.

Les trois colorants photochromes utilisés dans les exemples ci-après sont des dérivés naphtopyranes de la société James ROBINSON commercialisés sous les dénominations :

| | | |
|---|---|---|
| Reversacol | 195 | Réf. 1 |
| Reversacol | 208 | Réf. 2 |
| Reversacol | 306 | Réf. 3 |

Leurs ΔE respectifs ont été appréciés selon le protocole décrit dans la description. Le tableau I rend compte des écarts de teinte (ΔE) obtenus après 2 minutes d'exposition.

| Photochromes | ΔE |
|---|---|
| Réf. 1 | 39 |
| Réf. 2 | 6 |
| Réf. 3 | 34 |

Chacune de ces trois matières colorantes photochromes est formulée dans une base rouge à lèvres de composition comme suit et destinée à figurer une des deux composantes d'un produit rouge à lèvres bicouche.

Formulation de rouge à lèvres de base :

- octyl-2 dodecanol 0,5 %
- hectorite modifiée par chlorure de di-stearyl di-methyl ammonium 0,6 %
- lanoline liquide 27,2 %
- cire microcristalline 10,5 %
- cire d'abeille polyglycérolée (3 moles) 4,2 %
- lanoline acétylée 6,7 %
- huile d'arara (esters d'acide oléique) 13,5 %
- cire de lanoline oxypropylénée (5 op) 6,7 %
- érucate d'oléyle 13,5 %
- triglycérides d'acides oléique-linoléique-linolénique 1,7 %
- triglycérides d'acides palmitique-oléique-linoléique 13,5 %
- hyaluronate de sodium 0,1 %
- conservateurs 0,1 %
- vitamine 0,5 %
- filtre UV 0,7 %
- pigments 7 %
- matière colorante photochrome considérée 2 %

Pour chacune des trois formulations ainsi obtenues, on superpose à une couche de celle-ci, une couche de formulation gloss comprenant 95 % de polybutylène et 5 % d'hélicone commercialisés par la société WACKER.

Dès l'exposition au soleil, le produit appliqué change significativement de couleur pour chacun des 3 produits testés. L'exposition au soleil révèle les hélicones. Ce changement de couleur est particulièrement marqué avec les formulations comprenant respectivement les matières colorantes Réf. 1 et 3.

## Revendications

1. Produit cosmétique comprenant au moins une première et une seconde composition, la première composition comprenant dans un premier milieu physiologiquement acceptable au moins une première matière colorante et la seconde composition comprenant dans un second milieu physiologiquement acceptable, au moins une seconde matière colorante, ladite première matière colorante étant photochrome, et ladite seconde matière colorante étant au moins un agent de coloration goniochromatique.

2. Produit selon la revendication 1, **caractérisé en ce que** la matière colorante photochrome est organique.

3. Produit selon la revendication 2, **caractérisé en ce que** la matière colorante photochrome est de type naphtopyrane.

4. Produit selon la revendication 1, 2 ou 3, **caractérisé en ce que** ladite première matière colorante est au moins un 2H-naphto-[2,1-b]-pyrane de formule (I) ou un 3H-naphto-[2,1-b]-pyrane de formule (II) :

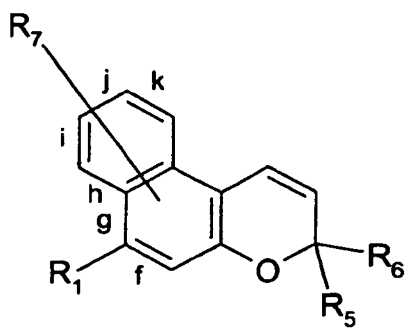

(I)

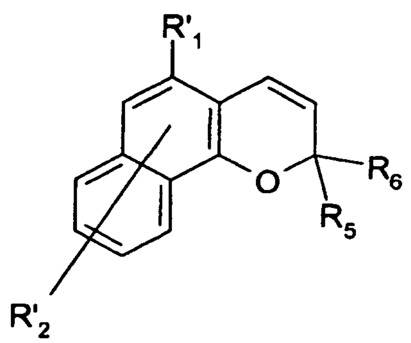

(II)

dans lesquelles :

- $R_1$ représente :

  - (i) un atome d'hydrogène;
  - (ii) un groupement hydrocarboné ayant 1 à 30 atomes de carbone, linéaires ramifiés, ou cycliques, saturés ou insaturés, éventuellement comportant 1 à 5 hétéroatomes choisis parmi N, O, S, Si et P, et/ou éventuellement halogéné ou perhalogéné;
  - (iii) un cycle hydrocarboné formé avec l'une des liaisons "f" ou "gh" et le radical $R_7$; ou
  - (iv) un groupement choisi parmi $-COOR_4$, $-C(O)NR_2R_3$, $-NR_2R_3$, $-OR_4$ et - $SR_4$, dans lequel :

- $R_2$ et $R_3$ soit représentent, indépendamment l'un de l'autre, un groupement hydrocarboné ayant 1 à 20 atomes de carbone, linéaire, ramifié ou cyclique, saturé ou insaturé, éventuellement comportant 1 à 5 hétéroatomes choisis parmi N, O, S, Si et P,
soit pris ensemble avec l'atome d'azote auquel ils sont reliés, forment un hétérocycle hydrocarboné, saturé ou insaturé, comportant 3 à 10 atomes de carbone et éventuellement 1 à 5 autres hétéroatomes choisis parmi N, O, S, Si et P, ledit cycle étant éventuellement substitué par au moins un radical hydrocarboné ayant 1 à 20 atomes de carbone, linéaire, ramifié ou cyclique, saturé ou insaturé, comportant éventuellement 1 à 5 hétéroatomes choisis parmi N, O, S, Si et P;
- $R_4$ représente un groupement hydrocarboné ayant 1 à 20 atomes de carbone, linéaire, ramifié ou cyclique, saturé ou insaturé, éventuellement halogéné ou perhalogéné, et/ou éventuellement comportant 1 à 5 hétéroatomes choisis parmi N, O, S, Si et P;
- $R_5$ et $R_6$ représentent, indépendamment l'un de l'autre, un groupement choisi parmi :

  - (i) les groupements aminoaryles cycliques saturés de formule (IIA) ou (IIB) :

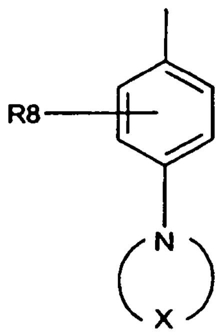

(IIa)

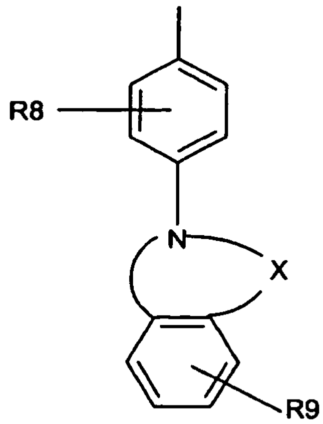

(IIb)

dans lesquelles le cycle comportant N et X est un cycle saturé qui comprend au total 3 à 30 atomes, inclus l'azote, le reste étant des atomes de carbone et/ou des hétéroatomes choisis parmi O, S, Si, P et/ou des groupements choisis parmi -NH et -NR avec R représentant un radical hydrocarboné ayant 1 à 20 atomes de carbone, linéaire, ramifié ou cyclique, saturé ou insaturé, éventuellement comportant 1 à 5 hétéroatomes choisis parmi N, O, S, Si et P;
  - (ii) les groupements indolinoaryles de formule (III) :

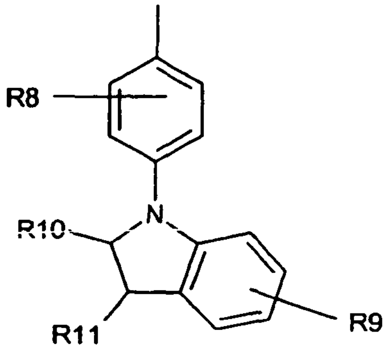

(III)

dans laquelle $R_{10}$ et $R_{11}$ représentent, indépendamment l'un de l'autre, un groupement choisi parmi (i) les groupements hydrocarbonés ayant 1 à 30 atomes de carbone, linéaires, ramifiés ou cycliques, saturés ou insaturés, éventuellement comportant 1 à 5 hétéroatomes choisis parmi N, O, S, Si et P, et/ou éventuellement halogéné ou perhalogéné, (ii) les atomes d'halogène, (iii) les groupements -CN (nitrile), -COOH (carboxylate), $-NO_2$ (nitro); (iv) un atome d'hydrogène; (v) un groupement choisi parmi $-C(O)NR_2R_3$, $-NR_2R_3$, $-OR_4$ ou $-SR_4$ avec $R_2$, $R_3$ et $R_4$ ayant les significations données ci-dessus; (vi) les radicaux $R_{10}$ et $R_{11}$ pouvant former ensemble un cycle hydrocarboné saturé ou insaturé ayant au total 5 à 8 atomes (incluant les atomes du cycle indoline), lesdits atomes étant choisis parmi C, O, S et/ou NR avec R représentant H ou un radical hydrocarboné ayant 1 à 20 atomes de carbone, linéaire, ramifié ou cyclique, saturé ou insaturé, éventuellement comportant 1 à 5 hétéroatomes choisis parmi N, O, S, Si et P,

- (iii) les groupements de formule (IV) :

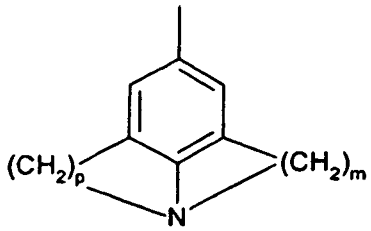

(IV)

dans laquelle m et p sont, indépendamment l'un de l'autre, des entiers allant de 2à5;

- (iv) les groupements aminoaryles cycliques insaturés de formules (VA), (VB) ou (VC) :

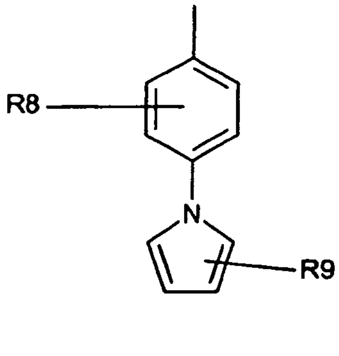

(VA)

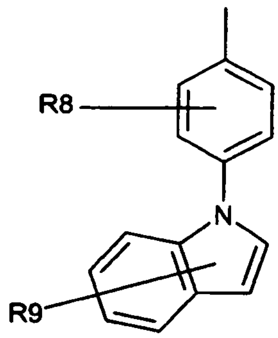

(VB)

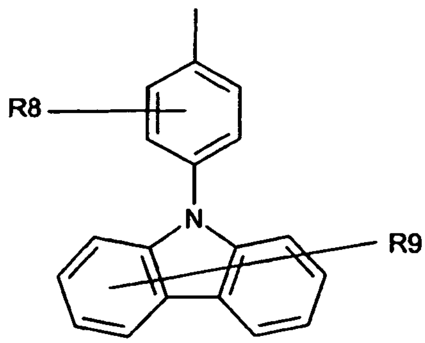

(VC)

dans lesquelles $R_8$ et $R_9$, représentent, indépendamment l'un de l'autre, un groupement choisi parmi (i) les groupements hydrocarbonés ayant 1 à 30 atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné, (ii) les atomes d'halogène, (iii) les groupements -CN (nitrile), -COOH (carboxylate), $-NO_2$ (nitro); (iv) un atome d'hydrogène; (v) un groupement choisi parmi $-C(O)NR_2R_3$, $-NR_2R_3$, $-OR_4$ ou $-SR_4$ avec $R_2$, $R_3$ et $R_4$ ayant les significations données ci-dessus;
- (v) un groupement hydrocarboné ayant 1 à 30 atomes de carbone, linéaire, ramifié ou cyclique, saturé ou insaturé, éventuellement comportant 1 à 5 hétéroatomes choisis parmi N, O, S, Si et P; et notamment un groupement choisi parmi $-C_6H_4-CONR_2R_3$, $-C_6H_4-NR_2R_3$ et $-C_6H_4-OR_4$ avec $R_2$, $R_3$ et $R_4$ ayant les significations données ci-dessus;

- $R_7$ représente un groupement choisi parmi :

- (i) les groupements hydrocarbonés ayant 1 à 30 atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné,
- (ii) les atomes d'halogène,
- (iii) les groupements -CN (nitrile), -COOH (carboxylate), $-NO_2$ (nitro); -N=N- (azo); =NH (imino); $-CONH_2$ (amide);
- (iv) un atome d'hydrogène;
- (v) un groupement choisi parmi $-C(O)NR_2R_3$, $-NR_2R_3$, $-OR_4$ ou $-SR_4$ avec $R_2$, $R_3$ et $R_4$ ayant les significations données ci-dessus;
- (vi) le radical $R_7$ pouvant en outre former, avec l'une des liaisons "i", "j ", "k", ou "g,h" prises avec le radical $R_1$, ou "f" prise avec le radical $R_1$, un cycle hydrocarboné saturé ayant au total 3 à 8 atomes de carbone,

éventuellement comportant 1 à 5 hétéroatomes choisis parmi N, O, S, Si et P;

- $R'_1$ représente un groupement choisi parmi :

  - (i) un atome d'hydrogène;
  - (ii) un groupement hydrocarboné ayant 1 à 30 atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné,
  - (iii) un groupement choisi parmi $-C(O)NR_2R_3$, $-NR_2R_3$, $-OR_4$ et $-SR_4$, avec $R_2$, $R_3$ et $R_4$ ayant les significations données ci-dessus;

- $R'_2$ représente un groupement choisi parmi :

  - (i) les groupements hydrocarbonés ayant 1 à 30 atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné,
  - (ii) les atomes d'halogène,
  - (iii) les groupements -CN (nitrile), -COOH (carboxylate), $-NO_2$ (nitro); -N=N- (azo); =NH (imino); $-CONH_2$ (amide);
  - (iv) un atome d'hydrogène;
  - (v) un groupement choisi parmi $-C(O)NR_2R_3$, $-NR_2R_3$, $-OR_4$ ou $-SR_4$ avec $R_2$, $R_3$ et $R_4$ ayant les significations données ci-dessus.

**5.** Produit selon l'une quelconque des revendications précédentes, **caractérisé en ce que** ladite première matière colorante répond à la formule (Ia) ou (IIa) suivante :

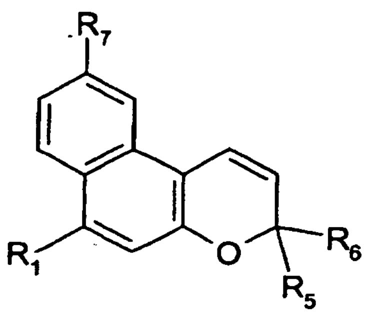

(Ia)

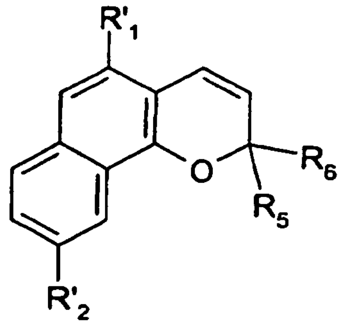

(IIa)

dans lesquelles $R_1$, $R_5$, $R_6$, $R_7$, $R'_1$ et $R'_2$ sont tels que définis en revendication 4.

**6.** Produit selon la revendication 4 ou 5, **caractérisé en ce que** $R_1$ représente un atome d'hydrogène; un cycle hydrocarboné avec l'une des liaisons "f" ou "gh" et le radical $R_7$; ou un groupement choisi parmi $-COOR_4$, $-NR_2R_3$, $-OR_4$ et $-SR_4$, dans lequel :

- $R_2$ et $R_3$ soit peuvent représenter, indépendamment l'un de l'autre, un groupement hydrocarboné ayant 1 à 20 atomes de carbone, linéaire, ramifié ou cyclique, saturé ou insaturé, éventuellement comportant 1 à 5 hétéroatomes choisis parmi N, O, S, Si et P,
soit pris ensemble avec l'atome d'azote auquel ils sont reliés, peuvent former un hétérocycle hydrocarboné, saturé ou insaturé, comportant 3 à 10 atomes de carbone et éventuellement 1 à 5 autres hétéroatomes choisis parmi N, O, S, Si et P, ledit cycle étant éventuellement substitué par au moins un radical hydrocarboné ayant 1 à 20 atomes de carbone, linéaire, ramifié ou cyclique, saturé ou insaturé, comportant éventuellement 1 à 5 hétéroatomes choisis parmi N, O, S, Si et P;
et/ou

- $R_4$ représente un groupement hydrocarboné ayant 1 à 20 atomes de carbone, linéaire, ramifié ou cyclique, saturé ou insaturé, éventuellement halogéné ou perhalogéné, et/ou éventuellement comportant 1 à 5 hétéroatomes choisis parmi N, O, S, Si et P.

et/ou

- $R_5$ et $R_6$ peuvent représenter, indépendamment l'un de l'autre, un groupement choisi parmi :

  - les groupements aminoaryles cycliques saturés de formule (IIA) ou (IIB) :

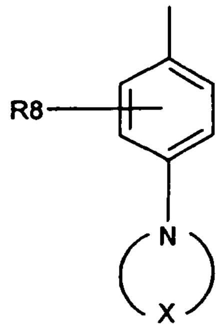

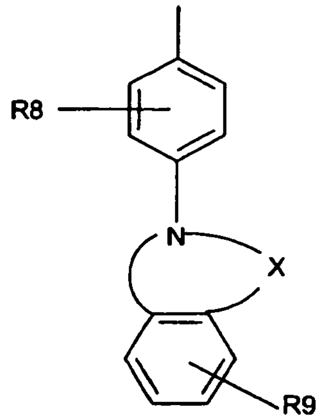

(IIA) (IIB)

dans lesquelles le cycle comportant N et X est un cycle saturé qui comprend au total 3 à 30 atomes inclus l'azote, le reste étant des atomes de carbone et/ou des hétéroatomes choisis parmi O, S, Si, P et/ou des groupements choisis parmi -NH et -NR avec R représentant un radical hydrocarboné ayant 1 à 20 atomes de carbone, linéaire, ramifié ou cyclique, saturé ou insaturé, éventuellement comportant 1 à 5 hétéroatomes choisis parmi N, O, S, Si et P;

  - un groupement hydrocarboné ayant 1 à 30 atomes de carbone, linéaire, ramifié ou cyclique, saturé ou insaturé, éventuellement comportant 1 à 5 hétéroatomes choisis parmi N, O, S, Si et P; et notamment un groupement choisi parmi $-C_6H_4-CONR_2R_3$, $-C_6H_4-NR_2R_3$ et $-C_6H_4-OR_4$ avec $R_2$, $R_3$ et $R_4$ ayant les significations données ci-dessus;

**7.** Produit selon l'une quelconque des revendications 4, 5, ou 6, **caractérisé en ce que** ladite première matière colorante répond à la formule (I) ou (Ia) pour lesquelles :

- $R_1$ représente l'hydrogène; ou un groupement -COOR avec R étant un radical hydrocarboné saturé ayant 1 à 12, notamment 1 à 6 atomes de carbone ou un groupement

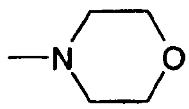

et/ou

- $R_5$ et $R_6$ représentent, indépendamment l'un de l'autre, soit (i) un groupement de formule (IIA) :

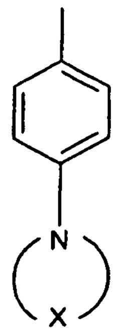

(IIA)

dans laquelle le cycle comportant N et X est un cycle saturé qui comprend au total 4 à 7 atomes, inclus l'azote, et en particulier un groupement de formule:

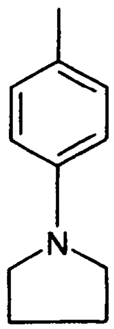

ou

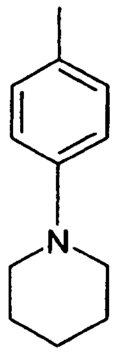

ou

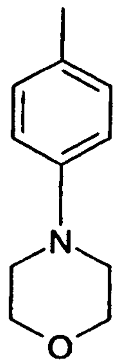

soit (ii) un groupement hydrocarboné ayant 5 à 14 atomes de carbone, linéaire, ramifié ou cyclique, saturé ou insaturé, éventuellement comportant 1 à 2 hétéroatomes choisis parmi N, O ou S;
et/ou
- $R_7$ représente un atome d'hydrogène ou un groupement $-NR_2R_3$, avec $R_2$ et $R_3$ représentant, indépendamment l'un de l'autre, un groupement hydrocarboné saturé, linéaire ou ramifié, ayant 1 à 12 atomes de carbone.

**8.** Produit selon l'une quelconque des revendications 4 à 7, **caractérisé en ce que** ladite première matière colorante répond à la formule (II) ou (IIa) pour lesquelles :

- $R'_1$ représente l'hydrogène ou un groupement -COOR avec R étant un radical hydrocarboné saturé ayant 1 à 12 atomes de carbone;
et/ou
- $R_5$ et $R_6$ représentent, indépendamment l'un de l'autre, soit (i) un groupement de formule (IIA) :

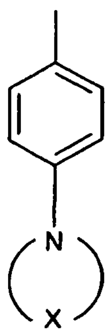

(IIA)

dans laquelle le cycle comportant N et X est un cycle saturé qui comprend au total 4 à 7 atomes, inclus l'azote, soit (ii) un groupement hydrocarboné ayant 5 à 14 atomes de carbone, linéaire, ramifié ou cyclique, saturé ou insaturé, éventuellement comportant 1 à 2 hétéroatomes choisis parmi N, O ou S;
et/ou

- $R'_2$ représente l'hydrogène, ou un groupement -NR'R" avec R' et R", identiques ou différents, représentant un groupement hydrocarboné saturé, linéaire ou ramifié, ayant 1 à 12 atomes de carbone.

**9.** Produit selon l'une quelconque des revendications précédentes, **caractérisé en ce que** ladite première matière colorante est choisie parmi

- le 3,3-di(4-méthoxyphényl)-6-morpholino-3H-naphto[2,1-b]pyrane
- le 3-phényl-3-(4-morpholinophényl)-6-morpholino-3H-naphto[2,1-b]pyrane
- le 3-phényl-3-(4-pipéridinophényl)-6-morpholino-3H-naphto[2,1-b]pyrane
- le 3-phényl-3-(4-pipéridinophényl)-6-carboxyméthyl-9-N-diméthyl-3H-naphto[2,1-b]pyrane
- le 2-phényl-2-(4-piperidinophényl)-5-carboxyméthyl-9-N-diméthyl-2H-naphto[1,2-b]pyrane et
- leurs mélanges.

**10.** Produit selon l'une quelconque des revendications précédentes, **caractérisé en ce que** ladite première(s) matière (s) colorante(s) est (sont) présente(s) à raison de 0,001 à 20 % en poids par rapport au poids total de la composition la ou les contenant.

**11.** Produit selon l'une quelconque des revendications précédentes, **caractérisé en ce qu'**il comprend au moins deux matières colorantes distinctes dans une même composition.

**12.** Produit selon l'une quelconque des revendications précédentes, **caractérisé en ce que** l'agent de coloration goniochromatique est présent à raison de 0,1 à 60 % en poids par rapport au poids total de la composition le contenant.

**13.** Produit selon l'une quelconque des revendications précédentes, **caractérisé en ce que** l'agent de coloration goniochromatique est choisi parmi les structures multicouches interférentielles et les agents de coloration à cristaux liquides.

**14.** Produit selon la revendication 13, **caractérisé en ce que** l'agent de coloration goniochromatique à structure multicouche interférentielle comporte au moins deux couches, chaque couche, indépendamment ou non de la (ou les) autre(s) couche(s), étant réalisée à partir d'au moins un matériau choisi dans le groupe constitué par les matériaux suivants : $MgF_2$, $CeF_3$, ZnS, ZnSe, Si, $SiO_2$, Ge, Te, $Fe_2O_3$, Pt, Va, $Al_2O_3$, MgO, $Y_2O_3$, $S_2O_3$, SiO, $HfO_2$, $ZrO_2$, $CeO_2$, $Nb_2O_5$, $Ta_2O_5$, $TiO_2$, Ag, Al, Au, Cu, Rb, Ti, Ta, W, Zn, $MoS_2$, cryolithe, alliages, polymères et leurs associations.

**15.** Produit selon la revendication 13, **caractérisé en ce que** l'agent de coloration à cristaux liquides est obtenu par polymérisation d'un mélange de monomères comprenant :

- a) au moins un premier monomère A de formule (I) Y1-A1-M1-A2-Y2
dans laquelle

  - i) Y1 et Y2 , identiques ou différents, représentent un groupe acrylate ou méthacrylate ;
  - ii) A1 et A2, identiques ou différents, représentent un groupement de formule $-CnH_2n-$, dans laquelle n est un nombre entier allant de 1 à 20 ;
  - iii) M1 désigne un groupement de formule générale (I') $-R_1-X_1-R_2-X_2-R_3-X_3-R_4-$, dans laquelle $R_1$ et $R_4$ désignent -O-, $R_2$ et $R_3$ désignent -COO-,

et $X_1$,$X_2$,$X_3$ sont un groupement 1,4 phénylène, le groupe carbonyl -CO- respectivement de $R_2$ et de $R_3$ étant lié respectivement au groupement $X_1$, $X_3$ ,
et
- b) au moins un deuxième monomère B chiral de formule (II) V1- W1- Z- W2- V2, dans laquelle

  - i) V1 désigne un groupement acrylate ou méthacrylate, et V2 désigne un groupement alkyle en $C_1$-$C_{20}$, un alkoxy en $C_1$-$C_{20}$, un alkoxy($C_1$-$C_{20}$)carbonyl, -OH ;

ii) W1 représente un groupement divalent de formule -X'1-CO-O-,

W2 représente un groupement divalent de formule -O-CO-X'1-, dans lesquelles X'1 désigne un groupe 1,4-phénylène, et Z désigne un groupement chiral à deux liaisons, issu du groupement dianhydrohexite, en particulier un radical divalent de formule :

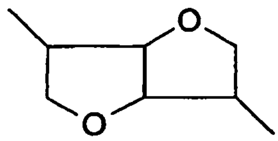

**16.** Produit selon la revendication 15, **caractérisé en ce que** l'agent de coloration à cristaux liquides est obtenu à partir d'un mélange de monomères comprenant de 70 à 99 % en poids de monomère A et de 1 à 30 % en poids de monomère B, par rapport au poids total de monomère A et de monomère B.

**17.** Produit selon la revendication 13, 15 ou 16, **caractérisé en ce que** l'agent de coloration à cristaux liquides est présent à raison de 0,01 % à 99 % en poids par rapport au poids total de la composition le contenant.

**18.** Produit selon l'une quelconque des revendications précédentes, **caractérisé en ce que** la première et/ou seconde composition(s) comprennent en outre au moins une matière colorante non goniochromatique et distincte de la première matière colorante.

**19.** Produit selon la revendication 18, **caractérisé en ce que** ladite matière colorante est choisie parmi les colorants monochromes hydrosolubles ou liposolubles, pigments, particules réfléchissantes, nacres et leurs mélanges.

**20.** Produit selon l'une quelconque des revendications précédentes, **caractérisé en ce que** la première et/ou seconde composition(s) comprennent en outre au moins un colorant monochrome liposoluble ou hydrosoluble.

**21.** Produit selon la revendication 20, **caractérisé en ce que** ledit colorant est présent à raison de 0,001 à 15 % en poids par rapport au poids total de la composition le contenant.

**22.** Produit selon l'une quelconque des revendication précédentes, **caractérisé en ce** la première et/ou seconde composition(s) comprennent en outre au moins un pigment.

**23.** Produit selon la revendication 22, **caractérisé en ce que** ledit pigment est présent à raison de 0,01 à 25 % en poids par rapport au poids total de la composition le contenant.

**24.** Produit selon l'une quelconque des revendications précédentes, **caractérisé en ce que** la première et/ou seconde composition(s) comprennent en outre au moins une nacre.

**25.** Produit selon la revendication 24, **caractérisé en ce que** ladite nacre est présente à raison de 0,01 à 20 % en poids par rapport au poids total de la composition la contenant.

**26.** Produit selon l'une quelconque des revendications précédentes, **caractérisé en ce que** la première et/ou seconde composition(s) comprennent en outre au moins des particules réfléchissantes.

**27.** Produit selon la revendication 26, **caractérisé en ce que** lesdites particules réfléchissantes sont présentes à raison de 0,1 % à 20 % en poids par rapport au poids total de la composition les contenant.

**28.** Produit selon l'une quelconque des revendications précédentes, **caractérisé en ce que** la première et/ou la seconde composition(s) comprennent au moins une phase huileuse.

**29.** Produit selon la revendication 28**caractérisé en ce que** la phase huileuse comprend une ou plusieurs huiles, polaires ou apolaires, volatiles ou non volatiles, et de préférence, hydrocarbonées.

**30.** Produit selon la revendication 28 ou 29, **caractérisé en ce que** la phase huileuse comprend de 5 à 100 % en poids d'huile(s) polaire(s) par rapport au poids total de ladite phase huileuse.

**31.** Produit selon la revendication 28, 29 ou 30, **caractérisé en ce que** la phase huileuse comprend de 5 à 100 % en poids d'huile(s) apolaire(s) par rapport au poids total de ladite phase huileuse.

**32.** Produit selon l'une quelconque des revendications 28 à 31, **caractérisé en ce que** les huiles peuvent être choisies parmi, les huiles d'origine animale, végétale, minérale ou synthétique, volatiles ou non et leurs mélanges.

**33.** Produit selon la revendication 32, **caractérisé en ce qu'**elles sont choisies parmi les huiles animales ou végétales, les esters et les éthers de synthèse notamment d'acides gras, les alcools gras, les hydrocarbures linéaires ou ramifiés, d'origine minérale ou synthétique les glycérides et leurs mélanges.

**34.** Produit selon l'une quelconque des revendications 28 à 33, **caractérisé en ce que** la phase huileuse est telle que la ou les première(s) matière(s) colorante(s) y sont solubles.

**35.** Produit selon l'une quelconque des revendications 28 à 34, **caractérisée en ce que** la phase huileuse est présente à raison de 1 à 99 % en poids par rapport au poids total de la composition la contenant.

**36.** Produit selon l'une quelconque des revendications précédentes, **caractérisé en ce que** la première et/ou la seconde composition(s) comprennent en outre de 0,1 à 50 % en poids d'un corps gras autre qu'une huile par rapport au poids total de la composition le contenant.

**37.** Produit selon l'une quelconque des revendications précédentes, **caractérisé en ce que** la première et/ou la seconde composition(s) sont anhydres.

**38.** Produit selon l'une quelconque des revendications 1 à 37, **caractérisé en ce que** la première et/ou la seconde composition(s) comprennent au moins une phase aqueuse.

**39.** Produit selon la revendication 38, **caractérisé en ce que** ladite phase aqueuse comprend de 0,1 à 14 % en poids, d'un monoalcool en $C_2$-$C_6$ par rapport au poids total de ladite phase aqueuse.

**40.** Produit selon l'une quelconque des revendications précédentes, **caractérisé en ce que** la première et/ou la seconde composition(s) comprennent en outre au moins un tensioactif.

**41.** Produit selon la revendication 40, **caractérisé en ce que** ledit tensioactif est présent en une quantité variant de 0,01 à 30 % en poids par rapport au poids total de la composition le contenant.

**42.** Produit selon l'une quelconque des revendications précédentes, **caractérisé en ce que** la première et/ou la seconde composition(s) comprennent en outre au moins un agent épaississant.

**43.** Produit selon la revendication 42, **caractérisé en ce que** ledit agent épaississant est présent à raison de 0,01 à 6 % en poids par rapport au poids total de la composition le contenant.

**44.** Produit selon l'une quelconque des revendications précédentes, **caractérisé en ce que** la première et/ou la seconde composition(s) comprennent en outre au moins un polymère filmogène.

**45.** Produit selon la revendication 44, **caractérisé en ce que** ledit polymère filmogène est présent à raison de 0,01 à 40 % en poids par rapport au poids total de la composition le contenant.

**46.** Produit selon l'une quelconque des revendications précédentes, **caractérisé en ce que** la première et/ou la seconde composition(s) comprennent en outre au moins une charge.

**47.** Produit selon la revendication 46, **caractérisé en ce que** ladite charge est présente à raison de 0,01 à 60 % en poids par rapport au poids total de la composition la contenant.

**48.** Procédé de maquillage de la peau, des lèvres et/ou des phanères comprenant l'application d'au moins un produit selon l'une quelconque des revendications précédentes sur la peau, les lèvres et/ou les phanères.

**49.** Procédé selon la revendication 48, **caractérisé en ce que** l'on applique en une première couche une première composition comprenant dans un premier milieu physiologiquement acceptable au moins ladite première matière colorante étant photochrome, puis l'on applique, sur tout ou partie de ladite première couche, une couche de la seconde composition comprenant, dans un second milieu physiologiquement acceptable, au moins un agent de coloration goniochromatique.

**50.** Kit de maquillage comprenant un produit selon l'une quelconque des revendications 1 à 47.

**51.** Kit selon la revendication 50, **caractérisé en ce qu'**il contient des moyens d'application de la première et de la seconde composition sur la peau, les lèvres et/ou les phanères.

**52.** Kit selon la revendication 50 ou 51, **caractérisé en ce qu'**il contient des moyens d'application choisis parmi les pinceaux, brosses, stylos, crayons, feutres, plumes, éponges et des mousses.

**53.** Kit selon l'une quelconque des revendications 50 à 52, **caractérisé en ce que** les première et seconde compositions sont conditionnées dans des articles de conditionnement distincts.

**Claims**

**1.** Cosmetic product comprising at least a first composition and a second composition, the first composition comprising at least one first dye in a first physiologically acceptable medium, and the second composition comprising at least one second dye in a second physiologically acceptable medium, the said first dye being photochromic and the said second dye being at least one goniochromatic colouring agent.

**2.** Product according to claim 1, **characterized in that** the photochromic dye is organic.

**3.** Product according to claim 2, **characterized in that** the photochromatic dye is naphthopyran type.

**4.** Product according to claim 1, 2 or 3, **characterized in that** the said first dye is at least one 2H-naphtho[2,1-b]pyran of formula (I) or a 3H-naphtho[2,1-b]pyran of formula (II):

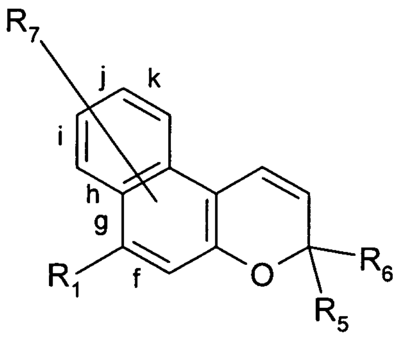

(I)

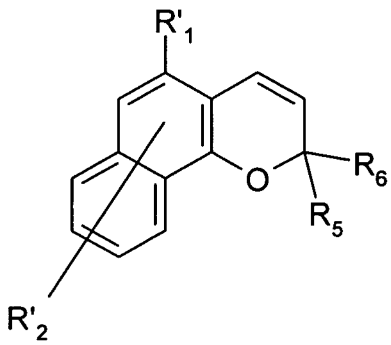

(II)

in which:

- $R_1$ represents:

  - (i) a hydrogen atom;
  - (ii) a linear, branched or cyclic, saturated or unsaturated hydrocarbon-based group containing from 1 to 30carbon atoms, optionally comprising 1 to 5 hetero atoms chosen from N, O, S, Si and P, and/or optionally halogenated or perhalogenated;
  - (iii) a hydrocarbon-based ring formed with one of the bonds "f" or "gh" and the radical $R_7$; or

- (iv) a group chosen from $-COOR_4$, $-C(O)NR_2R_3$, $-NR_2R_3$, $-OR_4$ and $-SR_4$, in which:

- $R_2$ and $R_3$ either represent, independently of each other, a linear, branched or cyclic, saturated or unsaturated hydrocarbon-based group containing 1 to 20 carbon atoms, optionally comprising 1 to 5 hetero atoms chosen from N, O, S, Si and P,
or, taken together with the nitrogen atom to which they are attached, form a saturated or unsaturated hydrocarbon-based heterocycle containing 3 to 10 carbon atoms and optionally 1 to 5 other hetero atoms chosen from N, O, S, Si and P, the said ring optionally being substituted with at least one linear, branched or cyclic, saturated or unsaturated hydrocarbon-based radical containing 1 to 20 carbon atoms, optionally comprising 1 to 5 hetero atoms chosen from N, O, S, Si and P;
- $R_4$ represents a linear, branched or cyclic, saturated or unsaturated hydrocarbon-based group containing 1 to 20 carbon atoms, which is optionally halogenated or perhalogenated and/or optionally comprising 1 to 5 hetero atoms chosen from N, O, S, Si and P;
- $R_5$ and $R_6$ represent, independently of each other, a group chosen from:

- (i) the saturated cyclic aminoaryl groups of formula (IIA) or (IIB):

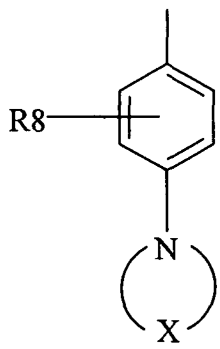

(IIA)

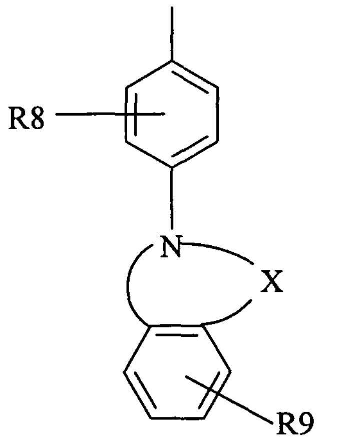

(IIB)

in which the ring comprising N and X is a saturated ring containing in total 3 to 30 atoms, including the nitrogen, the remainder being carbon atoms and/or hetero atoms chosen from O, S, Si and P and/or groups chosen from -NH and -NR with R representing a linear, branched or cyclic, saturated or unsaturated hydrocarbon-based radical containing 1 to 20 carbon atoms, optionally comprising 1 to 5 hetero atoms chosen from N, O, S, Si and P;
- (ii) the indolinoaryl groups of formula (III):

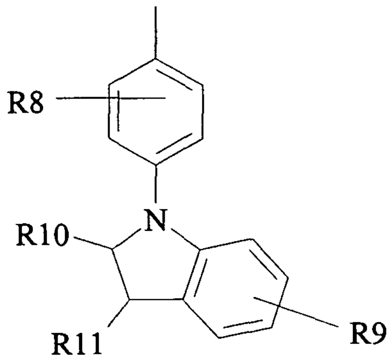

(III)

in which $R_{10}$ and $R_{11}$ represent, independently of each other, a group chosen from (i) linear, branched or cyclic, saturated or unsaturated hydrocarbon-based groups containing 1 to 30 carbon atoms, optionally comprising 1 to 5 hetero atoms chosen from N, O, S, Si and P, and/or optionally halogenated or perhalogenated; (ii) halogen atoms, (iii) - CN (nitrile), -COOH (carboxylate) or $-NO_2$ (nitro) groups; (iv) a hydrogen atom; (v) a group chosen from $-C(O)NR_2R_3$, $-NR_2R_3$, $-OR_4$ and $-SR_4$ with $R_2$, $R_3$ and $R_4$ having the meanings given above; (vi) the radicals $R_{10}$ and $R_{11}$ together possibly forming a saturated or unsaturated hydrocarbon-based ring containing in total 5 to 8 atoms (including the atoms of the indoline ring), the said atoms being chosen from C, O, S and/or NR with R representing H or a linear, branched or cyclic, saturated or unsaturated hydrocarbon-based radical containing 1 to 20 carbon atoms, optionally comprising 1 to 5 hetero atoms chosen from N, O, S, Si and P,

- (iii) the groups of formula (IV):

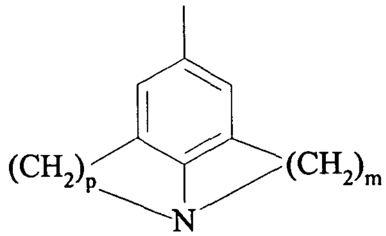

(IV)

in which m and p are, independently of each other, integers ranging from 2 to 5;

- (iv) the unsaturated cyclic aminoaryl groups of formula (VA), (VB) or (VC):

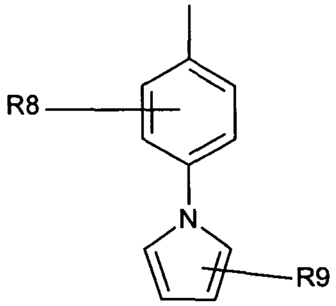

(VA)

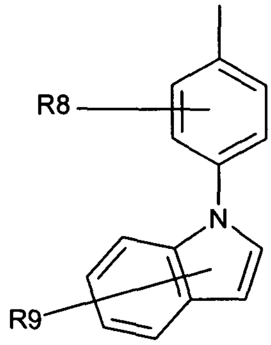

(VB)

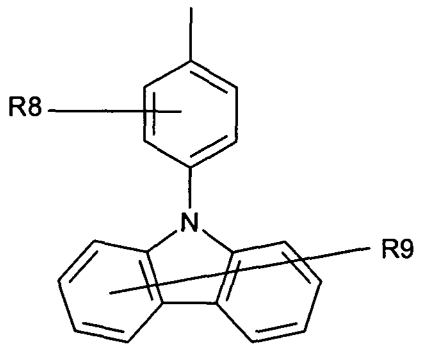

(VC)

in which $R_8$ and $R_9$ represent, independently of each other, a group chosen from (i) linear, branched or cyclic, saturated or unsaturated hydrocarbon-based groups containing 1 to 30 carbon atoms, optionally comprising 1 to 5 hetero atoms chosen from N, O, S, Si and P, and/or optionally halogenated or perhalogenated; (ii) halogen atoms; (iii) - CN (nitrile), -COOH (carboxylate) or $-NO_2$ (nitro) groups; (iv) a hydrogen atom; (v) a group chosen from $-C(O)NR_2R_3$, $-NR_2R_3$, $-OR_4$ and $-SR_4$ with $R_2$, $R_3$ and $R_4$ having the meanings given above;

- (v) a linear, branched or cyclic, saturated or unsaturated hydrocarbon-based group containing 1 to 30 carbon atoms, optionally comprising 1 to 5 hetero atoms chosen from N, O, S, Si and P; and especially a group chosen from $-C_6H_4-CONR_2R_3$, $-C_6H_4-NR_2R_3$ and $-C_6H_4-OR_4$ with $R_2$, $R_3$ and $R_4$ having the meanings given above;

- $R_7$ represents a group chosen from:

  - (i) linear, branched or cyclic, saturated or unsaturated hydrocarbon-based groups containing 1 to 30 carbon atoms, optionally comprising 1 to 5 hetero atoms chosen from N, O, S, Si and P, and/or optionally halogenated or perhalogenated;
  - (ii) halogen atoms;
  - (iii) -CN (nitrile), -COOH (carboxylate), $-NO_2$ (nitro), -N=N- (azo), =NH (imino) or $-CONH_2$ (amide) groups;
  - (iv) a hydrogen atom;
  - (v) a group chosen from $-C(O)NR_2R_3$, $-NR_2R_3$, $-OR_4$ and $-SR_4$ with $R_2$, $R_3$ and $R_4$ having the meanings given above;
  - (vi) the radical $R_7$ also possibly forming, with one of the bonds "i", "j", "k" or "g,h" taken with the radical $R_1$, or "f" taken with the radical $R_1$, a saturated hydrocarbon-based ring containing in total 3 to 8 carbon atoms, optionally comprising 1 to 5 hetero atoms chosen from N, O, S, Si and P;

- $R'_1$ represents a group chosen from:

  - (i) a hydrogen atom;
  - (ii) a linear, branched or cyclic, saturated or unsaturated hydrocarbon-based group containing 1 to 30 carbon atoms, optionally comprising 1 to 5 hetero atoms chosen from N, O, S, Si and P, and/or optionally halogenated or perhalogenated;
  - (iii) a group chosen from $-C(O)NR_2R_3$, $-NR_2R_3$, $-OR_4$ and $-SR_4$, with $R_2$, $R_3$ and $R_4$ having the meanings given above;

- $R'_2$ represents a group chosen from:

  - (i) linear, branched or cyclic, saturated or unsaturated hydrocarbon-based groups containing 1 to 30 carbon atoms, optionally comprising 1 to 5 hetero atoms chosen from N, O, S, Si and P, and/or optionally halogenated or perhalogenated;
  - (ii) halogen atoms;
  - (iii) -CN (nitrile), -COOH (carboxylate), $-NO_2$ (nitro), -N=N- (azo), =NH (imino) or $-CONH_2$ (amide) groups;
  - (iv) a hydrogen atom;
  - (v) a group chosen from $-C(O)NR_2R_3$, $-NR_2R_3$, $-OR_4$ and $-SR_4$, with $R_2$, $R_3$ and $R_4$ having the meanings given above.

**5.** Product according to any one of the preceding claims, **characterized in that** the said first dye corresponds to formula (Ia) or (IIa) below:

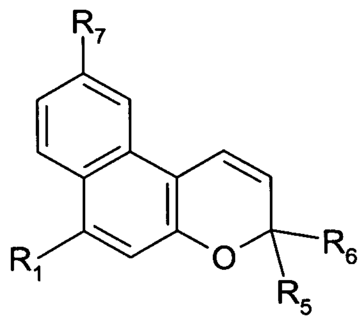

(Ia)

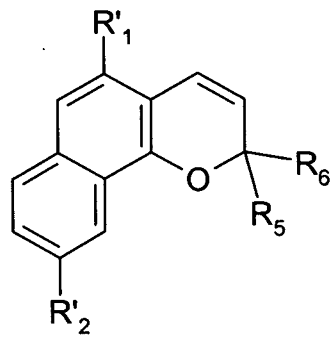

(IIa)

in which $R_1$, $R_5$, $R_6$, $R_7$, $R'_1$ and $R'_2$ are as defined in claim 4.

**6.** Product according to claim 4 or 5, **characterized in that** $R_1$ represents a hydrogen atom; a hydrocarbon-based ring with one of the bonds "f" or "gh" and the radical $R_7$; or a group chosen from $-COOR_4$, $-NR_2R_3$, $-OR_4$ and $-SR_4$, in which:

- $R_2$ and $R_3$ either may represent, independently of each other, a linear, branched or cyclic, saturated or unsaturated hydrocarbon-based group containing 1 to 20 carbon atoms, optionally comprising 1 to 5 hetero atoms chosen from N, O, S, Si and P,
or, taken together with the nitrogen atom to which they are attached, may form a saturated or unsaturated hydrocarbon-based heterocycle containing 3 to 10 carbon atoms and optionally 1 to 5 other hetero atoms chosen from N, O, S, Si and P, the said ring optionally being substituted with at least one linear, branched or cyclic, saturated or unsaturated hydrocarbon-based radical containing 1 to 20 carbon atoms, optionally comprising 1 to 5 hetero atoms chosen from N, O, S, Si and P;
and/or
- $R_4$ represents a linear, branched or cyclic, saturated or unsaturated hydrocarbon-based group containing 1 to 20 carbon atoms, optionally halogenated or perhalogenated, and/or optionally comprising 1 to 5 hetero atoms chosen from N, O, S, Si and P;

and/or
- $R_5$ and $R_6$ may represent, independently of each other, a group chosen from:

- the saturated cyclic aminoaryl groups of formula (IIA) or (IIB):

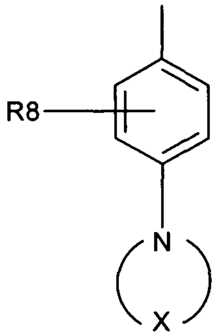

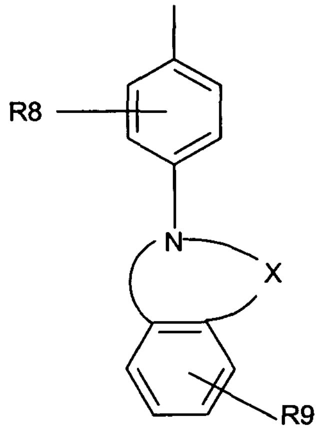

(IIA) (IIB)

in which the ring comprising N and X is a saturated ring which contains in total 3 to 30 atoms, including nitrogen, the rest being carbon atoms and/or hetero atoms chosen from O, S, Si and P and/or groups chosen from -NH and -NR with R representing a linear, branched or cyclic, saturated or unsaturated hydrocarbon-based radical containing 1 to 20carbon atoms, optionally comprising 1 to 5 hetero atoms chosen from N, O, S, Si and P;

- a linear, branched or cyclic, saturated or unsaturated hydrocarbon-based group containing 1 to 30 carbon atoms, optionally comprising 1 to 5 hetero atoms chosen from N, O, S, Si and P; and especially a group chosen from $-C_6H_4-CONR_2R_3$, $-C_6H_4-NR_2R_3$ and $-C_6H_4-OR_4$ with $R_2$, $R_3$ and $R_4$ having the meanings given above.

**7.** Product according to any one of claims 4, 5 or 6, **characterized in that** the said first dye corresponds to formula (I) or (Ia) for which:

- $R_1$ represents hydrogen; or a group -COOR with R being a saturated hydrocarbon-based radical containing 1 to 12 carbon atoms or a group

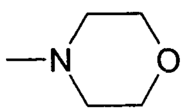

and/or
- $R_5$ and $R_6$ represent, independently of each other, either (i) a group of formula (IIA):

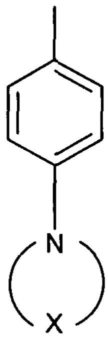

(IIA)

in which the ring comprising N and X is a saturated ring containing in total 4 to 7 atoms, including nitrogen, and in particular a group of formula:

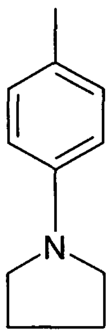

or

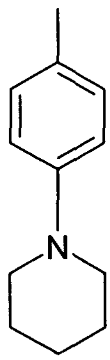

or

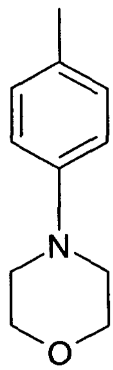

.

or (ii) a linear, branched or cyclic, saturated or unsaturated hydrocarbon-based group containing 5 to 14 carbon atoms, optionally comprising 1 or 2 hetero atoms chosen from N, O or S;
and/or
$R_7$ represents a hydrogen atom or a group $-NR_2R_3$, with $R_2$ and $R_3$ representing, independently of each other, a linear or branched, saturated hydrocarbon-based group containing 1 to 12 carbon atoms.

**8.** Product according to any one of claims 4 to 7, **characterized in that** the said first dye corresponds to formula (II) or (IIa) for which:

- $R'_1$ represents hydrogen or a group -COOR with R being a saturated hydrocarbon-based radical containing 1 to 12 carbon atoms;
and/or
- $R_5$ and $R_6$ represent, independently of each other, either (i) a group of formula (IIA):

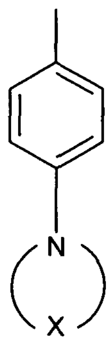

(IIA)

in which the ring comprising N and X is a saturated ring containing in total 4 to 7 atoms, including nitrogen,

or (ii) a linear, branched or cyclic, saturated or unsaturated hydrocarbon-based group containing 5 to 14 carbon atoms, optionally comprising 1 or 2 hetero atoms chosen from N, O and S;
and/or
- $R'_2$ represents hydrogen or a group -NR'R", with R' and R", which may be identical or different, representing a linear or branched, saturated hydrocarbon-based group containing 1 to 12 carbon atoms.

9. Product according to any one of the preceding claims, **characterized in that** the said first dye is chosen from:

- 3,3-di(4-methoxyphenyl)-6-morpholino-3H-naphtho[2,1-b]pyran
- 3-phenyl-3-(4-morpholinophenyl)-6-morpholino-3H-naphtho[2,1-b]pyran
- 3-phenyl-3-(4-piperidinophenyl)-6-morpholino-3H-naphtho[2,1-b]pyran
- 3-phenyl-3-(4-piperidinophenyl)-6-carboxymethyl-9-N-dimethyl-3H-naphtho[2,1-b]pyran
- 2-phenyl-2-(4-piperidinophenyl)-5-carboxymethyl-9-N-dimethyl-2H-naphtho[1,2-b]pyran, and
- mixtures thereof.

10. Product according to any one of the preceding claims, **characterized in that** the said first dye(s) is(are) present in a proportion of from 0.001 % to 20% by weight relative to the total weight of the composition containing it or them.

11. Product according to any one of the preceding claims, **characterized in that** it comprises at least two different first dyes in the same composition.

12. Product according to any one of the preceding claims, **characterized in that** the goniochromatic colouring agent is present in a proportion of from 0.1% to 60% by weight, relative to the total weight of the composition containing it.

13. Product according to any one of the preceding claims, **characterized in that** the goniochromatic colouring agent is chosen from multilayer interference structures and liquid-crystal colouring agents.

14. Product according to claim 13, **characterized in that** the goniochromatic colouring agent of multilayer interference structure comprises at least two layers, each layer, optionally independently of the other layer(s), being made from at least one material chosen from the group consisting of the following materials: MgF2, CeF3, ZnS, ZnSe, Si, SiO2, Ge, Te, Fe2O3, Pt, Va, Al203, MgO, Y2O3, S2O3, SiO, HfO2, ZrO2, Ce02, Nb2O5, Ta2O5, Ti02, Ag, Al, Au, Cu, Rb, Ti, Ta, W, Zn, MoS2, cryolite, alloys and polymers, and combinations thereof.

15. Product according to claim 13, **characterized in that** the liquid-crystal colouring agent is obtained by polymerization of a monomer mixture comprising:

- a) at least a first monomer A of formula (I) Y1-A1-M1-A2-Y2
in which

- i) Y1 and Y2, which may be identical or different, represent an acrylate or methacrylate group;
- ii) A1 and A2, which may be identical or different, represent a group of formula $-CnH_2n-$, in which n is an integer ranging from 1 to 20;
- iii) M1 denotes a group of general formula (I') $-R_1-X_1-R_2-X_2-R_3-X_3-R_4-$, in which $R_1$ and $R_4$ denote -O-, and $R_2$ and $R_3$ denote -COO-,

and $X_1$, $X_2$ and $X_3$ are a 1,4-phenylene group, the carbonyl group -CO- of $R_2$ and of $R_3$, respectively, being linked to the group $X_1$ or $X_3$, respectively, and
- b) at least a second monomer B, which is chiral, of formula (II) V1- W1- Z- W2- V2, in which

- i) V1 denotes an acrylate or methacrylate group, and V2 denotes a $C_1$-$C_{20}$ alkyl, $C_1$-$C_{20}$ alkoxy, ($C_1$-$C_{20}$) alkoxycarbonyl or -OH group;
- ii) W1 represents a divalent group of formula -X'1-CO-O-,

W2 represents a divalent group of formula -O-CO-X'1-,
in which X'1 denotes a 1,4-phenylene group,
and Z denotes a chiral group containing two bonds, derived from the dianhydrohexite group, in particular a divalent radical of formula:

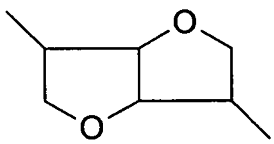

16. Product according to claim 15, **characterized in that** the liquid-crystal colouring agent is obtained from a monomer mixture comprising from 70% to 99% by weight of monomer A and from 1% to 30% by weight of monomer B relative to the total weight of monomer A and of monomer B.

17. Product according to claim 13, 15 or 16, **characterized in that** the liquid-crystal colouring agent is present in a proportion of from 0.01% to 99% by weight, relative to the total weight of the composition containing it.

18. Product according to any one of the preceding claims, **characterized in that** the first and/or second composition (s) also comprise at least one non-goniochromatic dye that is different from the first dye.

19. Product according to claim 18, **characterized in that** the said dye is chosen from water-soluble or liposoluble monochromatic dyes, pigments, reflective particles and nacres, and mixtures thereof.

20. Product according to any one of the preceding claims, **characterized in that** the first and/or second composition (s) also comprise(s) at least one liposoluble or water-soluble monochromatic dye.

21. Product according to claim 20, **characterized in that** said dye is present in a proportion of from 0.001% to 15% by weight relative to the total weight of the composition containing it.

22. Product according to any one of the preceding claims, **characterized in that** the first and/or second composition (s) also comprise(s) at least one pigment.

23. Product according to claim 22, **characterized in that** said pigment is present in a proportion of from 0.01% to 25% by weight relative to the total weight of the composition containing it.

24. Product according to any one of the preceding claims, **characterized in that** the first and/or second composition (s) also comprise(s) at least one nacre.

25. Product according to claim 24, **characterized in that** said nacre is present in a proportion of from 0.01% to 20% by weight relative to the total weight of the composition containing it.

26. Product according to any one of the preceding claims, **characterized in that** the first and/or second composition (s) also comprise(s) at least reflective particles.

27. Product according to claim 26, **characterized in that** said reflective particles are present in a proportion of from 0.1 % to 20% by weight relative to the total weight of the composition containing them.

28. Product according to any one of the preceding claims, **characterized in that** the first and/or second composition (s) comprise(s) at least one oily phase.

29. Product according to claim 28, **characterized in that** the oily phase comprises one or more polar or apolar, volatile or non-volatile and, preferably, hydrocarbon-based oils.

30. Product according to claim 28 or 29, **characterized in that** the oily phase comprises from 5% to 100% by weight of polar oil(s) relative to the total weight of the said oily phase.

31. Product according to claim 28, 29 or 30, **characterized in that** the oily phase comprises from 5% to 100% by weight of apolar oil(s) relative to the total weight of the said oily phase.

32. Product according to any one of claims 28 to 31, **characterized in that** the oils may be chosen from volatile or non-volatile oils of animal, plant, mineral or synthetic origin, and mixtures thereof.

**33.** Product according to claim 32, **characterized in that** the oils are chosen from animal or plant oils, synthetic esters and ethers, especially of fatty acids, fatty alcohols, linear or branched hydrocarbons of mineral or synthetic origin, and glycerides, and mixtures thereof.

**34.** Product according to any one of claims 28 to 33, **characterized in that** the oily phase is such that the first dye(s) is(are) soluble therein.

**35.** Product according to any one of claims 28 to 34, **characterized in that** the oily phase is present in a proportion of from 1% to 99% by weight relative to the total weight of the composition containing it.

**36.** Product according to any one of the preceding claims, **characterized in that** the first and/or second composition (s) also comprise(s) from 0.1% to 50% by weight of a fatty substance other than an oil, relative to the total weight of the composition containing it.

**37.** Product according to any one of the preceding claims, **characterized in that** the first and/or second composition (s) is(are) anhydrous.

**38.** Product according to any one of claims 1 to 37, **characterized in that** the first and/or second composition(s) comprise (s) at least one aqueous phase.

**39.** Product according to claim 38, **characterized in that** the said aqueous phase comprises from 0.1 % to 14% by weight of a $C_2$-$C_6$ monoalcohol relative to the total weight of the said aqueous phase.

**40.** Product according to any one of the preceding claims, **characterized in that** the first and/or second composition (s) also comprise(s) at least one surfactant.

**41.** Product according to claim 40, **characterized in that** said surfactant is present in an amount ranging from 0.01% to 30% by weight relative to the total weight of the composition containing it.

**42.** Product according to any one of the preceding claims, **characterized in that** the first and/or second composition (s) also comprise(s) at least one thickener.

**43.** Product according to claim 42, **characterized in that** said thickener is present in a proportion of from 0.01% to 6% by weight relative to the total weight of the composition containing it.

**44.** Product according to any one of the preceding claims, **characterized in that** the first and/or second composition (s) also comprise(s) at least one film-forming polymer.

**45.** Product according to claim 44, **characterized in that** said film forming polymer is present in a proportion of from 0.01 % to 40% by weight relative to the total weight of the composition containing it.

**46.** Product according to any one of the preceding claims, **characterized in that** the first and/or second composition (s) also comprise(s) at least one filler.

**47.** Product according to claim 46, **characterized in that** said filler is present in a proportion of from 0.01% to 60% by weight relative to the total weight of the composition containing it.

**48.** Process for making up the skin, the lips and/or the integuments, comprising the application of at least one product according to any one of the preceding claims to the skin, the lips and/or the integuments.

**49.** Process according to claim 48, **characterized in that** a first composition comprising at least the said first dye being photochromic in a first physiologically acceptable medium is applied, as a first coat, and a coat of the second composition comprising at least one goniochromatic colouring agent in a second physiologically acceptable medium is then applied over all or part of the said first coat.

**50.** Makeup kit comprising a product according to any one of claims 1 to 47.

**51.** Kit according to claim 50, **characterized in that** it contains means for applying the first and the second composition

to the skin, the lips and/or the integuments.

52. Kit according to claim 50 or 51, **characterized in that** it contains application means chosen from small or large brushes, pens, pencils, felts, nibs, sponges and foams.

53. Kit according to any one of claims 50 to 52, **characterized in that** the first and second compositions are packaged in separate packaging articles.

## Patentansprüche

1. Kosmetisches Produkt, umfassend mindestens eine erste und eine zweite Zusammensetzung, wobei die erste Zusammensetzung in einem ersten physiologisch annehmbaren Medium mindestens einen ersten Farbstoff umfasst und die zweite Zusammenfassung in einem zweiten physiologisch annehmbaren Medium mindestens einen zweiten Farbstoff umfasst, wobei der erste Farbstoff fotochrom ist und der zweite Farbstoff mindestens ein goniochromatisches Färbungsmittel ist.

2. Produkt nach Anspruch 1, **dadurch gekennzeichnet, dass** der fotochrome Farbstoff organisch ist.

3. Produkt nach Anspruch 2, **dadurch gekennzeichnet, dass** der fotochrome Farbstoff vom Typ Naphtopyran ist.

4. Produkt nach Anspruch 1, 2 oder 3, **dadurch gekennzeichnet, dass** der erste Farbstoff mindestens ein 2H-Naphto-[2,1-b]-pyran der Formel (I) oder ein 3H-Naphto-[2,1-b]-pyran der Formel (II) ist:

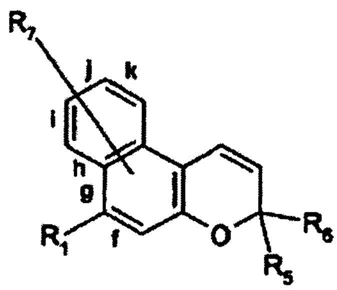

(I)

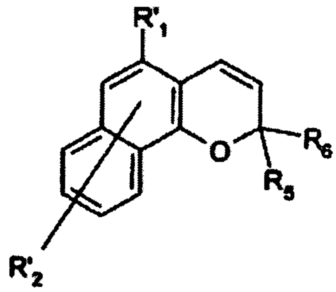

(II)

in denen:

- $R_1$ darstellt:

  - (i) ein Wasserstoffatom;
  - (ii) eine Kohlenwasserstoffgruppe mit 1 bis 30 Kohlenstoffatomen, linear, verzweigt oder cyclisch, gesättigt oder ungesättigt, gegebenenfalls mit 1 bis 5 Heteroatomen, die aus N, O, S, Si und P ausgewählt sind, und/oder gegebenenfalls halogeniert oder perhalogeniert;
  - (iii) einen mit einer der Bindungen "f" oder "gh" und dem Rest $R_7$ gebildeten Kohlenwasserstoffring; oder
  - (iv) eine Gruppe, die aus $-COOR_4$, $-C(O)NR_2R_3$, $-NR_2R_3$, $-OR_4$ und $-SR_4$ ausgewählt ist, in der:

- $R_2$ und $R_3$ entweder unabhängig voneinander eine lineare, verzweigte oder cyclische, gesättigte oder ungesättigte Kohlenwasserstoffgruppe mit 1 bis 20 Kohlenstoffatomen gegebenenfalls mit 1 bis 5 Heteroatomen, die aus N, O, S, Si und P ausgewählt sind, darstellen
oder zusammen mit dem Stickstoffatom, an das sie gebunden sind, einen gesättigten oder ungesättigten Kohlenwasserstoffheterocyclus mit 3 bis 10 Kohlenstoffatomen und gegebenenfalls 1 bis 5 anderen Heteroatomen, die aus N, O, S, Si und P ausgewählt sind, bilden, wobei dieser Ring gegebenenfalls mit mindestens einem linearen, verzweigten oder cyclischen, gesättigten oder ungesättigten Kohlenwasserstoffrest substituiert ist,

der gegebenenfalls 1 bis 5 Heteroatome umfasst, die aus N, O, S, Si und P ausgewählt sind;

- $R_4$ eine lineare, verzweigte oder cyclische, gesättigte oder ungesättigte Kohlenwasserstoffgruppe mit 1 bis 20 Kohlenstoffatomen darstellt, die gegebenenfalls halogeniert oder perhalogeniert ist und/oder gegebenenfalls 1 bis 5 Heteroatome aufweist, die aus N, O, S, Si und P ausgewählt sind;
- $R_5$ und $R_6$ unabhängig voneinander eine Gruppe darstellen, die ausgewählt ist aus:

  - (i) den gesättigten cyclischen Aminoarylgruppen der Formel (IIA) oder (IIB):

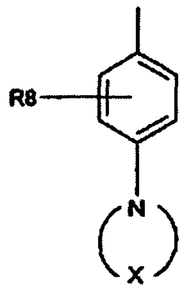

(IIa)

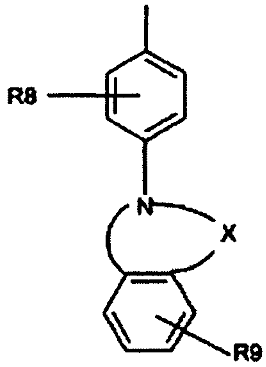

(IIb)

in denen der N und X aufweisende Ring ein gesättigter Ring ist, der insgesamt 3 bis 30 Atome einschließlich des Stickstoffs umfasst, wobei der Rest Kohlenstoffatome und/oder aus O, S, Si, P ausgewählte Heteroatome und/oder Gruppen sind, die aus -NH und -NR ausgewählt sind, wobei R einen linearen, verzweigten oder cyclischen, gesättigten oder ungesättigten Kohlenwasserstoffrest mit 1 bis 20 Kohlenstoffatomen darstellt, der gegebenenfalls 1 bis 5 Heteroatome umfasst, die aus N, O, S, Si und P ausgewählt sind;

- (ii) den Indolinoarylgruppen der Formel (III):

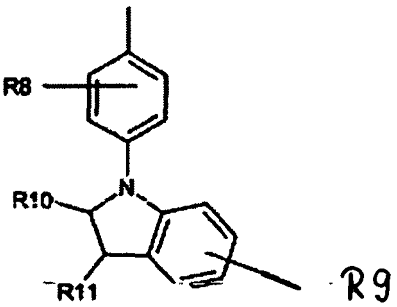

(III)

in der $R_{10}$ und $R_{11}$ unabhängig voneinander eine Gruppe darstellen, die ausgewählt ist aus: (i) den linearen, verzweigten oder cyclischen, gesättigten oder ungesättigten Kohlenwasserstoffgruppen mit 1 bis 30 Kohlenstoffatomen, die gegebenenfalls 1 bis 5 Heteroatome umfassen, die aus N, O, S, Si und P ausgewählt sind, und/oder gegebenenfalls halogeniert oder perhalogeniert, (ii) den Halogenatomen, (iii) den Gruppen -CN (Nitril), -COOH (Carboxylat), $-NO_2$(Nitro); (iv) einem Wasserstoffatom; (v) einer Gruppe, die aus $-C(O)NR_2R_3$, $-NR_2R_3$, $-OR_4$ oder $-SR_4$ ausgewählt ist, wobei $R_2$, $R_3$ und $R_4$ die oben angegebenen Bedeutungen haben; (vi) wobei die Reste $R_{10}$ und $R_{11}$ zusammen einen gesättigten oder ungesättigten Kohlenwasserstoffring mit insgesamt 5 bis 8 Atomen (die die Atome des Indolinrings einschließen) bilden können, wobei diese Atome aus C, O, S und/oder NR ausgewählt sind, wobei R H darstellt oder einen linearen, verzweigten oder cyclischen, gesättigten oder ungesättigten Kohlenwasserstoffrest mit 1 bis 20 Kohlenstoffatomen, der gegebenenfalls 1 bis 5 Heteroatome umfasst, die aus N, O, S, Si und P ausgewählt sind,

- (iii) den Gruppen der Formel (IV):

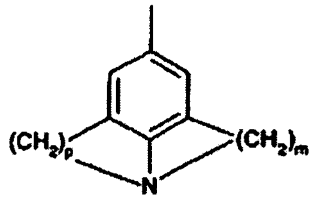

(IV)

in der m und p unabhängig voneinander ganze Zahlen von 2 bis 5 sind;
- (iv) den ungesättigten cyclischen Aminoarylgruppen der Formeln (VA), (VB) oder (VC):

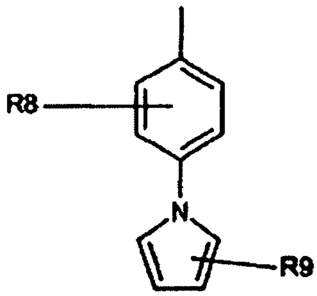

(VA)

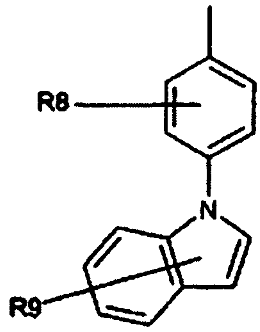

(VB)

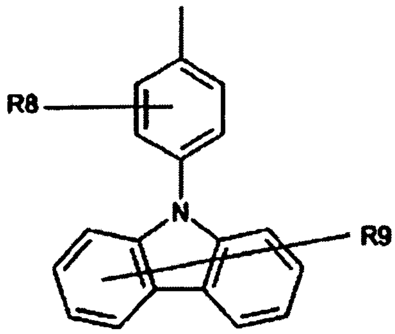

(VC)

in denen $R_8$ und $R_9$ unabhängig voneinander eine Gruppe darstellen, die ausgewählt ist aus: (i) den linearen, verzweigten oder cyclischen, gesättigten oder ungesättigten Kohlenwasserstoffgruppen mit 1 bis 30 Kohlenstoffatomen, die gegebenenfalls 1 bis 5 Heteroatome umfassen, die aus N, O, S, Si und P ausgewählt sind, und/oder gegebenenfalls halogeniert oder perhalogeniert, (ii) den Halogenatomen, (iii) den Gruppen -CN (Nitril), -COOH (Carboxylat), $-NO_2$ (Nitro); (iv) einem Wasserstoffatom; (v) einer Gruppe, die aus $-C(O)NR_2R_3$; $-NR_2R_3$, $-OR_4$ oder $-SR_4$ ausgewählt ist, wobei $R_2$, $R_3$ und $R_4$ die oben angegebenen Bedeutungen haben;

- (v) einer linearen, verzweigten oder cyclischen, gesättigten oder ungesättigten Kohlenwasserstoffgruppe mit 1 bis 30 Kohlenstoffatomen, die gegebenenfalls 1 bis 5 Heteroatome umfasst, die aus N, O, S, Si und P ausgewählt sind; und insbesondere einer Gruppe, die aus $-C_6H_4-CONR_2R_3$, $-C_6H_4-NR_2R_3$ und $-C_6H_4-OR_4$ ausgewählt ist, wobei $R_2$, $R_3$ und $R_4$ die oben angegebenen Bedeutungen haben;

- $R_7$ eine Gruppe darstellt, die ausgewählt ist aus:

  - (i) den linearen, verzweigten oder cyclischen, gesättigten oder ungesättigten Kohlenwasserstoffgruppen mit 1 bis 30 Kohlenstoffatomen, die gegebenenfalls 1 bis 5 Heteroatome umfassen, die aus N, O, S, Si und P ausgewählt sind, und/oder gegebenenfalls halogeniert oder perhalogeniert,
  - (ii) den Halogenatomen,
  - (iii) den Gruppen -CN (Nitril), -COOH (Carboxylat), $-NO_2$ (Nitro); -N=N- (Azo); =NH (Imino); $-CONH_2$ (Amid);
  - (iv) einem Wasserstoffatom;
  - (v) einer Gruppe, die aus $-C(O)NR_2R_3$, $-NR_2R_3$, $-OR_4$ oder $-SR_4$ ausgewählt ist, wobei $R_2$, $R_3$ und $R_4$ die oben angegebenen Bedeutungen haben;
  - (vi) wobei der Rest $R_7$ außerdem mit einer der Bindungen "i", "j", "k" oder "g,h", mit dem Rest $R_1$ genommen, oder "f", mit dem Rest $R_1$ genommen, einen gesättigten Kohlenwasserstoffring mit insgesamt 3 bis 8 Kohlenstoffatomen bilden kann, der gegebenenfalls 1 bis 5 Heteroatome umfasst, die aus N, O, S, Si und P ausgewählt sind;

- $R'_1$ eine Gruppe darstellt, die ausgewählt ist aus:

  - (i) einem Wasserstoffatom;
  - (ii) einer Kohlenwasserstoffgruppe mit 1 bis 30 Kohlenstoffatomen, linear, verzweigt oder cyclisch, gesättigt oder ungesättigt, gegebenenfalls mit 1 bis 5 Heteroatomen, die aus N, O, S, Si und P ausgewählt sind, und/oder gegebenenfalls halogeniert oder perhalogeniert,
  - (iii) einer Gruppe, die aus $-C(O)NR_2R_3$, $-NR_2R_3$, $-OR_4$ oder $-SR_4$ ausgewählt ist, wobei $R_2$, $R_3$ und $R_4$ die oben angegebenen Bedeutungen haben;

- $R'_2$ eine Gruppe darstellt, die ausgewählt ist aus:

  - (i) den linearen, verzweigten oder cyclischen, gesättigten oder ungesättigten Kohlenwasserstoffgruppen mit 1 bis 30 Kohlenstoffatomen, die gegebenenfalls 1 bis 5 Heteroatome umfassen, die aus N, O, S, Si und P ausgewählt sind, und/oder gegebenenfalls halogeniert oder perhalogeniert,
  - (ii) den Halogenatomen,
  - (iii) den Gruppen -CN (Nitril), -COOH (Carboxylat), $-NO_2$ (Nitro); -N=N- (Azo); =NH (Imino); $-CONH_2$ (Amid);
  - (iv) einem Wasserstoffatom;
  - (v) einer Gruppe, die aus $-C(O)NR_2R_3$, $-NR_2R_3$, $-OR_4$ oder $-SR_4$ ausgewählt ist, wobei $R_2$, $R_3$ und $R_4$ die oben angegebenen Bedeutungen haben.

**5.** Produkt nach einem der vorhergehenden Ansprüche, **dadurch gekennzeichnet, dass** der erste Farbstoff der folgenden Formel (Ia) oder (IIa) entspricht:

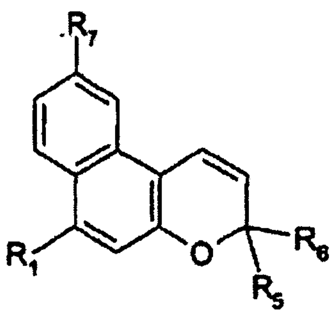

(Ia)

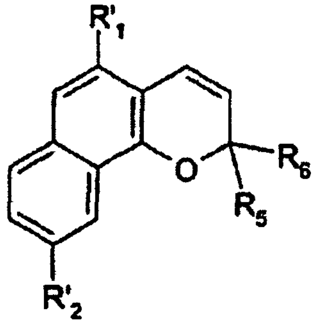

(IIa)

in denen $R_1$, $R_5$, $R_6$, $R_7$, $R'_1$ und $R'_2$ so sind, wie in Anspruch 4 definiert ist.

**6.** Produkt nach Anspruch 4 oder 5, **dadurch gekennzeichnet, dass** $R_1$ darstellt: ein Wasserstoffatom; einen Kohlenwasserstoffring mit einer der Bindungen "f" oder "gh" und dem Rest $R_7$; oder eine Gruppe, die aus $-CCOR_4$, $-NR_2R_3$, $-OR_4$ und $-SR_4$ ausgewählt ist, in der:

- $R_2$ und $R_3$ entweder unabhängig voneinander eine gesättigte oder ungesättigte, lineare, verzweigte oder cyclische Kohlenwasserstoffgruppe mit 1 bis 20 Kohlenstoffatomen darstellen können, die gegebenenfalls 1 bis 5 Heteroatome umfasst, die aus N, O, S, Si und P ausgewählt sind,
oder zusammen mit dem Stickstoffatom, an das sie gebunden sind, einen gesättigten oder ungesättigten Kohlenwasserstoffheterocyclus bilden können, der 3 bis 10 Kohlenstoffatome und gegebenenfalls 1 bis 5 andere Heteroatome umfasst, die aus N, O, S, Si und P ausgewählt sind, wobei dieser Ring gegebenenfalls mit mindestens einem gesättigten oder ungesättigten, linearen, verzweigten oder cyclischen Kohlenwasserstoffrest mit 1 bis 20 Kohlenstoffatomen substituiert ist, der gegebenenfalls 1 bis 5 Heteroatome umfasst, die aus N, O, S, Si und P ausgewählt sind;
und/oder
- $R_4$ eine gesättigte oder ungesättigte, lineare, verzweigte oder cyclische Kohlenwasserstoffgruppe darstellt, die gegebenenfalls halogeniert oder perhalogeniert ist und/oder gegebenenfalls 1 bis 5 Heteroatome umfasst, die aus N, O, S, Si und P ausgewählt sind;
und/oder
- $R_5$ und $R_6$ unabhängig voneinander eine Gruppe darstellen können, die ausgewählt ist aus:

  - den gesättigten cyclischen Aminoarylgruppen der Formel (IIA) oder (IIB):

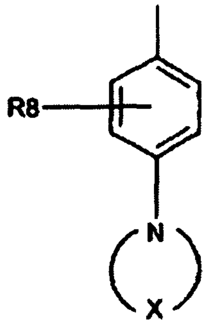

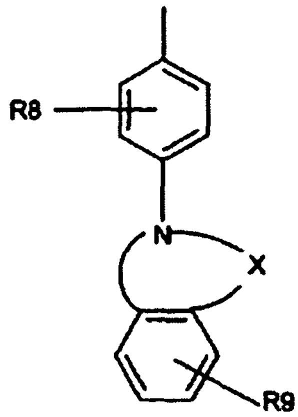

(IIA) (IIB)

in denen der N und X umfassende Ring ein gesättigter Ring ist, der insgesamt 3 bis 30 Atome einschließlich des Stickstoffs umfasst, wobei der Rest Kohlenstoffatome und/oder Heteroatome, die aus O, S, Si, P ausgewählt sind, und/oder Gruppen sind, die aus -NH und -NR ausgewählt sind, wobei R einen gesättigten oder ungesättigten linearen, verzweigten oder cyclischen Kohlenwasserstoffrest mit 1 bis 20 Kohlenstoffatomen darstellt, der gegebenenfalls 1 bis 5 Heteroatome umfasst, die aus N, O, S, Si und P ausgewählt sind;
- einer gesättigten oder ungesättigten, linearen, verzweigten oder cyclischen Kohlenwasserstoffgruppe mit 1 bis 30 Kohlenstoffatomen, die gegebenenfalls 1 bis 5 Heteroatome umfasst, die aus N, O, S, Si und P ausgewählt sind; und insbesondere einer Gruppe, die aus $-C_6H_4-CONR_2R_3$, $-C_6H_4NR_2R_3$ und $-C_6H_4-OR_4$ ausgewählt ist, wobei $R_2$, $R_3$ und $R_4$ die oben angegebenen Bedeutungen haben.

7. Produkt nach einem der Ansprüche 4, 5 oder 6, **dadurch gekennzeichnet, dass** der erste Farbstoff der Formel (I) oder (Ia) entspricht, bei denen:

- $R_1$ Wasserstoff darstellt; oder eine Gruppe -COOR, wobei R ein gesättigter Kohlenwasserstoffrest mit 1 bis 12, insbesondere 1 bis 6 Kohlenstoffatomen oder eine Gruppe

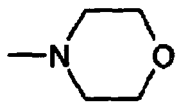

und/oder
- $R_5$ und $R_6$ unabhängig voneinander darstellen: entweder (i) eine Gruppe der Formel (IIA):

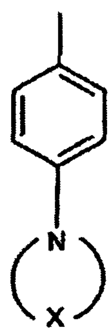

(IIA)

in der der N und X umfassende Ring ein gesättigter Ring ist, der insgesamt 4 bis 7 Atome einschließlich des Stickstoffs umfasst, und insbesondere eine Gruppe der Formel:

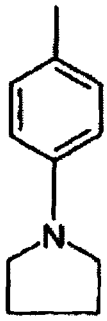 oder 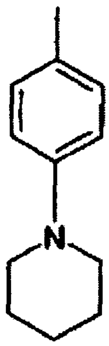 oder 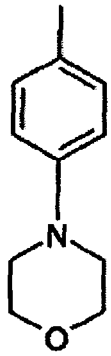

oder (ii) eine gesättigte oder ungesättigte lineare, verzweigte oder cyclische Kohlenwasserstoffgruppe mit 5 bis 14 Kohlenstoffatomen, die gegebenenfalls 1 bis 2 Heteroatome umfasst, die aus N, O oder S ausgewählt sind;
und/oder
- $R_7$ ein Wasserstoffatom oder eine Gruppe $-NR_2R_3$ darstellt, wobei $R_2$ und $R_3$ unabhängig voneinander eine gesättigte lineare oder verzweigte Kohlenwasserstoffgruppe mit 1 bis 12 Kohlenstoffatomen darstellen.

**8.** Produkt nach einem der Ansprüche 4 bis 7, **dadurch gekennzeichnet, dass** der erste Farbstoff der Formel (II) oder (IIa) entspricht, bei denen:

- $R'_1$ Wasserstoff oder eine Gruppe -COOR darstellt, wobei R ein gesättigter Kohlenwasserstoffrest mit 1 bis 12 Kohlenstoffatomen ist;
und/oder
- $R_5$ und $R_6$ unabhängig voneinander entweder (i) eine Gruppe der Formel (IIA):

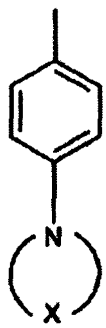

**(IIA)**

in der der N und X umfassende Ring ein gesättigter Ring ist, der insgesamt 4 bis 7 Atome einschließlich des Stickstoffs umfasst, oder
(ii) eine gesättigte oder ungesättigte lineare, verzweigte oder cyclische Kohlenwasserstoffgruppe mit 5 bis 14 Kohlenstoffatomen darstellen, die gegebenenfalls 1 bis 2 Heteroatome umfasst, die aus N, O oder S ausgewählt sind;
und/oder
- $R'_2$ Wasserstoff oder eine Gruppe -NR'R" darstellt, wobei R' und R", die gleich oder verschieden sind, eine gesättigte lineare oder verzweigte Kohlenwasserstoffgruppe mit 1 bis 12 Kohlenstoffatomen darstellen.

**9.** Produkt nach einem der vorhergehenden Ansprüche, **dadurch gekennzeichnet, dass** der erste Farbstoff ausgewählt ist aus

- 3,3-Di(4-methoxyphenyl)-6-morpholino-3H-naphtho[2,1-b]pyran

- 3-Phenyl-3-(4-morpholinophenyl)-6-morpholino-3H-naphto[2,1-b]pyran
- 3-Phenyl-3-(4-piperidinophenyl)-6-morpholino-3H-naphto[2,1-b]pyran
- 3-Phenyl-3-(4-piperidinophenyl)-6-carboxymethyl-9-N-dimethyl-3H-naphto[2,1-b]pyran
- 2-Phenyl-2-(4-piperidinophenyl)-5-carboxymethyl-9-N-dimethyl-2H-naphto[1,2-b]pyran und
- ihre Mischungen.

**10.** Produkt nach einem der vorhergehenden Ansprüche, **dadurch gekennzeichnet, dass** der bzw. die ersten Farbstoffe in einem Anteil von 0,001 bis 20 Gew.-% bezogen auf das Gesamtgewicht der ihn oder sie enthaltenden Zusammensetzung vorliegen.

**11.** Produkt nach einem der vorhergehenden Ansprüche, **dadurch gekennzeichnet, dass** es mindestens zwei verschiedene Farbstoffe in einer gleichen Zusammensetzung umfasst.

**12.** Produkt nach einem der vorhergehenden Ansprüche, **dadurch gekennzeichnet, dass** das goniochromatische Färbemittel in einem Anteil von 0,1 bis 60 Gew.-%, bezogen auf das Gesamtgewicht der es enthaltenden Zusammensetzung, vorliegt.

**13.** Produkt nach einem der vorhergehenden Ansprüche, **dadurch gekennzeichnet, dass** das goniochromatische Färbemittel aus den interferentiellen Multischichtstrukturen und den Flüssigkristall-Färbemitteln ausgewählt ist.

**14.** Produkt nach Anspruch 13, **dadurch gekennzeichnet, dass** das goniochromatische Färbemittel mit interferentieller Multischichtstruktur mindestens zwei Schichten umfasst, wobei jede Schicht unabhängig oder nicht unabhängig von der (oder den) anderen Schichten aus mindestens einem Werkstoff hergestellt ist, der aus der Gruppe ausgewählt ist, die aus den folgenden Stoffen besteht: $MgF_2$, $CeF_3$, ZnS, ZnSe, Si, $SiO_2$, $G_e$, Te, $Fe_2O_3$, Pt, Va, $Al_2O_3$, MgO, $Y_2O_3$, $S_2O_3$, SiO, $HfO_2$, $ZrO_2$, $CeO_2$, $Nb_2O_5$, $Ta_2O_5$, $TiO_2$, Ag, Al, Au, Cu, Rb, Ti, Ta, W, Zn, $MoS_2$, Cryolith, Legierungen, Polymere und ihre Kombinationen.

**15.** Produkt nach Anspruch 13, **dadurch gekennzeichnet, dass** das Flüssigkristall-Färbemittel durch Polymerisation einer Mischung von Monomeren erhalten wird, die umfasst:

- a) mindestens ein erstes Monomer A der Formel (I) Y1-A1-M1-A2-Y2,
in der

  - i) Y1 und Y2, die gleich oder verschieden sind, eine Acrylat- oder Methacrylatgruppe darstellen;
  - ii) A1 und A2, die gleich oder verschieden sind, eine Gruppe der Formel $-CnH_2n-$darstellen, in der n eine ganze Zahl von 1 bis 20 ist;
  - iii) M1 eine Gruppe der allgemeinen Formel (I') $-R_1-X_1-R_2-X_2-R_3-X_3-R_4-$ bezeichnet, in der $R_1$ und $R_4$ -O- bezeichnen, $R_2$ und $R_3$ -COO- bezeichnen,

und $X_1$, $X_2$, $X_3$ eine Gruppe 1,4-Phenylen sind, wobei die Carbonylgruppe -CO- von $R_2$ bzw. $R_3$ an die Gruppe $X_1$ bzw. $X_3$ gebunden ist,
und
- b) mindestens ein zweites chirales Monomer B der Formel (II) V1-W1-Z-W2-V2, in der

  - i) V1 eine Acrylat- oder Methacrylatgruppe bezeichnet und V2 eine $C_1$-$C_{20}$-Alkylgruppe, ein $C_1$-$C_{20}$-Alkoxy, ein Alkoxy($C_1$-$C_{20}$)carbonyl, -OH bezeichnet;
  ii) W1 eine zweiwertige Gruppe der Formel -X'1-CO-O- darstellt, W2 eine zweiwertige Gruppe der Formel -O-CO-X'1- darstellt, in der X'1 eine Gruppe 1,4-Phenylen bezeichnet, und Z eine chirale Gruppe mit zwei Bindungen bezeichnet, die aus der Dianhydrohexitgruppe hervorgeht, und zwar insbesondere einen zweiwertigen Rest der Formel:

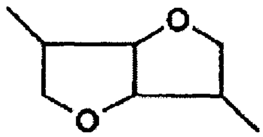

16. Produkt nach Anspruch 15, **dadurch gekennzeichnet, dass** das Flüssigkristall-Färbemittel aus einer Mischung von Monomeren erhalten ist, die 70 bis 99 Gew.-% Monomer A und 1 bis 30 Gew.-% Monomer B, bezogen auf das Gesamtgewicht von Monomer A und Monomer B, umfasst.

17. Produkt nach Anspruch 13, 15 oder 16, **dadurch gekennzeichnet, dass** das Flüssigkristall-Färbemittel in einem Anteil von 0,01 bis 99 Gew.-%, bezogen auf das Gesamtgewicht der es enthaltenden Zusammensetzung, vorliegt.

18. Produkt nach einem der vorhergehenden Ansprüche, **dadurch gekennzeichnet, dass** die erste und/oder die zweite Zusammensetzung außerdem mindestens einen nicht goniochromatischen Farbstoff umfassen, der von dem ersten Farbstoff verschieden ist.

19. Produkt nach Anspruch 18, **dadurch gekennzeichnet, dass** der Farbstoff aus den wasserlöslichen oder fettlöslichen monochromen Farbstoffen, Pigmenten, reflektierenden Teilchen, Perlmutt und ihren Mischungen ausgewählt ist.

20. Produkt nach einem der vorhergehenden Ansprüche, **dadurch gekennzeichnet, dass** die erste und/oder die zweite Zusammensetzung außerdem mindestens einen fettlöslichen oder wasserlöslichen monochromen Farbstoff umfassen.

21. Produkt nach Anspruch 20, **dadurch gekennzeichnet, dass** der Farbstoff in einem Anteil von 0,001 bis 15 Gew.-%, bezogen auf das Gesamtgewicht der ihn enthaltenden Zusammensetzung, vorliegt.

22. Produkt nach einem der vorhergehenden Ansprüche, **dadurch gekennzeichnet, dass** die erste und/oder die zweite Zusammensetzung außerdem mindestens ein Pigment umfassen.

23. Produkt nach Anspruch 22, **dadurch gekennzeichnet, dass** das Pigment in einem Anteil von 0,01 bis 25 Gew.-%, bezogen auf das Gesamtgewicht der es enthaltenden Zusammensetzung, vorliegt.

24. Produkt nach einem der vorhergehenden Ansprüche, **dadurch gekennzeichnet, dass** die erste und/oder die zweite Zusammensetzung außerdem mindestens ein Perlmutt umfassen.

25. Produkt nach Anspruch 24, **dadurch gekennzeichnet, dass** das Perlmutt in einem Anteil von 0,01 bis 20 Gew.-%, bezogen auf das Gesamtgewicht der es enthaltenden Zusammensetzung, vorliegt.

26. Produkt nach einem der vorhergehenden Ansprüche, **dadurch gekennzeichnet, dass** die erste und/oder die zweite Zusammensetzung außerdem mindestens reflektierende Teilchen umfassen.

27. Produkt nach Anspruch 26, **dadurch gekennzeichnet, dass** die reflektierenden Teilchen in einem Anteil von 0,1 bis 20 Gew.-%, bezogen auf das Gesamtgewicht der sie enthaltenden Zusammensetzung, vorliegen.

28. Produkt nach einem der vorhergehenden Ansprüche, **dadurch gekennzeichnet, dass** die erste und/oder die zweite Zusammensetzung mindestens eine ölige Phase umfassen.

29. Produkt nach Anspruch 28, **dadurch gekennzeichnet, dass** die ölige Phase ein oder mehrere polare oder apolare, flüchtige oder nichtflüchtige und vorzugsweise kohlenwasserstoffhaltige Öle umfasst.

30. Produkt nach Anspruch 28 oder 29, **dadurch gekennzeichnet, dass** die ölige Phase 5 bis 100 Gew.-% polares Öl bzw. polare Öle, bezogen auf das Gesamtgewicht der öligen Phase, umfasst.

31. Produkt nach Anspruch 28, 29 oder 30, **dadurch gekennzeichnet, dass** die ölige Phase 5 bis 100 Gew.-% apolares Öl bzw. apolare Öle, bezogen auf das Gesamtgewicht der öligen Phase, umfasst.

32. Produkt nach einem der Ansprüche 28 bis 31, **dadurch gekennzeichnet, dass** die Öle aus den flüchtigen oder nichtflüchtigen Ölen tierischen, pflanzlichen, mineralischen oder synthetischen Ursprungs und ihren Mischungen ausgewählt sein können.

33. Produkt nach Anspruch 32, **dadurch gekennzeichnet, dass** sie aus den tierischen oder pflanzlichen Ölen, den Estern und den Ethern der Synthese insbesondere von Fettsäuren, den Fettalkoholen, den linearen oder verzweigten Kohlenwasserstoffen mineralischen oder synthetischen Ursprungs, den Glyceriden und ihren Mischungen ausgewählt sind.

34. Produkt nach einem der Ansprüche 28 bis 33, **dadurch gekennzeichnet, dass** die ölige Phase so beschaffen ist, das der oder die ersten Farbstoffe darin löslich sind.

35. Produkt nach einem der Ansprüche 28 bis 34, **dadurch gekennzeichnet, dass** die ölige Phase in einem Anteil von 1 bis 99 Gew.-%, bezogen auf das Gesamtgewicht der sie enthaltenden Zusammensetzung, vorliegt.

36. Produkt nach einem der vorhergehenden Ansprüche, **dadurch gekennzeichnet, dass** die erste und/oder die zweite Zusammensetzung außerdem 0,1 bis 50 Gew.-% eines anderen Fetts als ein Öl, bezogen auf das Gesamtgewicht der es enthaltenden Zusammensetzung, umfassen.

37. Produkt nach einem der vorhergehenden Ansprüche, **dadurch gekennzeichnet, dass** die erste und/oder die zweite Zusammensetzung wasserfrei sind.

38. Produkt nach einem der Ansprüche 1 bis 37, **dadurch gekennzeichnet, dass** die erste und/oder die zweite Zusammensetzung mindestens eine wässrige Phase umfassen.

39. Produkt nach Anspruch 38, **dadurch gekennzeichnet, dass** die wässrige Phase 0,1 bis 14 Gew.-% eines einwertigen $C_2$-$C_6$-Alkohols, bezogen auf das Gesamtgewicht der wässrigen Phase, umfasst.

40. Produkt nach einem der vorhergehenden Ansprüche, **dadurch gekennzeichnet, dass** die erste und/oder die zweite Zusammensetzung außerdem mindestens ein Tensid umfassen.

41. Produkt nach Anspruch 40, **dadurch gekennzeichnet, dass** das Tensid in einer Menge vorliegt, die von 0,01 bis 30 Gew.-%, bezogen auf das Gesamtgewicht der es enthaltenden Zusammensetzung, variiert.

42. Produkt nach einem der vorhergehenden Ansprüche, **dadurch gekennzeichnet, dass** die erste und/oder die zweite Zusammensetzung außerdem mindestens ein Verdikkungsmittel umfassen.

43. Produkt nach Anspruch 42, **dadurch gekennzeichnet, dass** das Verdickungsmittel in einem Anteil von 0,01 bis 6 Gew.-%, bezogen auf das Gesamtgewicht der es enthaltenden Zusammensetzung, vorliegt.

44. Produkt nach einem der vorhergehenden Ansprüche, **dadurch gekennzeichnet, dass** die erste und/oder die zweite Zusammensetzung außerdem mindestens ein filmbildendes Polymer umfassen.

45. Produkt nach Anspruch 44, **dadurch gekennzeichnet, dass** das filmbildende Polymer in einem Anteil von 0,01 bis 40 Gew.-%, bezogen auf das Gesamtgewicht der es enthaltenden Zusammensetzung, vorliegt.

46. Produkt nach einem der vorhergehenden Ansprüche, **dadurch gekennzeichnet, dass** die erste und/oder die zweite Zusammensetzung außerdem mindestens einen Füllstoff umfassen.

47. Produkt nach Anspruch 46, **dadurch gekennzeichnet, dass** der Füllstoff in einem Anteil von 0,01 bis 60 Gew.-%, bezogen auf das Gesamtgewicht der ihn enthaltenden Zusammensetzung, vorliegt.

48. Verfahren zum Schminken der Haut, der Lippen und/oder der Hautanhangsorgane, umfassend das Auftragen mindestens eines Produkts nach einem der vorhergehenden Ansprüche auf die Haut, die Lippen und/oder die Hautanhangsorgane.

**49.** Verfahren nach Anspruch 48, **dadurch gekennzeichnet, dass** man in einer ersten Schicht eine erste Zusammensetzung aufträgt, die in einem ersten physiologisch annehmbaren Medium mindestens diesen ersten Farbstoff, der fotochrom ist, aufträgt und dann auf die ganze erste Schicht oder einen Teil davon eine Schicht der zweiten Zusammensetzung aufträgt, die in einem zweiten physiologisch annehmbaren Medium mindestens ein goniochromatisches Färbemittel umfasst.

**50.** Schminkset, umfassend ein Produkt nach einem der Ansprüche 1 bis 47.

**51.** Set nach Anspruch 50, **dadurch gekennzeichnet, dass** es Mittel zum Auftragen der ersten und der zweiten Zusammensetzung auf die Haut, die Lippen und/oder die Hautanhangsorgane enthält.

**52.** Set nach Anspruch 50 oder 51, **dadurch gekennzeichnet, dass** es Auftragmittel enthält, die aus Pinseln, Bürsten, Stiften, Liners, Filzen, Federn, Schwämmen und Schaumstoffen ausgewählt sind.

**53.** Set nach einem der Ansprüche 50 bis 52, **dadurch gekennzeichnet, dass** die erste und die zweite Zusammensetzung in getrennten Verpackungsartikeln verpackt sind.